# EUROPEAN PATENT SPECIFICATION

(11) **EP 3 793 523 B1**
(45) Date of publication and mention of the grant of the patent: **12.11.2025**
(21) Application number: 19728226.2
(22) Date of filing: 15.05.2019
(51) Int. Cl.: A61K 9/107, A61K 47/10, A61K 47/12, A61K 47/14, A61K 47/34, A61K 31/519, A61K 31/7068, C12Q 1/6886, G01N 33/574, G01N 33/68, A61P 35/00

(54) **FORMULATIONS AND METHODS FOR THE PREVENTION AND TREATMENT OF TUMOR METASTASIS AND TUMORIGENESIS**
FORMULIERUNGEN UND VERFAHREN ZUR PRÄVENTION UND BEHANDLUNG VON TUMORMETASTASEN UND TUMORGENESE
FORMULATIONS ET MÉTHODES DE PRÉVENTION ET DE TRAITEMENT DES MÉTASTASES TUMORALES ET DE LA TUMORIGENÈSE

(30) Priority: 15.05.2018 US 201862671964 P
(43) Date of publication of application: 24.03.2021
(73) Proprietor: The United States of America, as represented by the Secretary, Department of Health and Human Services, Bethesda, Maryland 20892-7788 (US)
(72) Inventor: RUDLOFF, Udo, Bethesda, Maryland 20892 (US); KOZLOV, Serguei, Bethesda, Maryland 20892 (US); MARUGAN, Juan Jose, Gaithersburg, Maryland 20878 (US); HUANG, Sui, Chicago, Illinois 60614 (US); PATNAIK, Samarjit, Gaithersburg, Maryland 20878 (US); BRAISTED, John C., Boyds, Maryland 20841 (US); SOUTHALL, Noel T., Potomac, Maryland 20854 (US); FERRER, Marc, Potomac, Maryland 20854 (US); DEXTRAS, Christopher, Grand Rapids, MI 49503 (US); HASLAM, John, Lawrence, Kansas 66047 (US); BALTEZOR, Michael, Shawnee, KS 66216 (US)
(74) Representative: Pfenning, Meinig & Partner mbB
(86) International application number: PCT/US2019/032461
(87) International publication number: WO 2019/222380

(56) References cited:
- WO-A2-2009/121069
- US-A1- 2010 136 105
- US-B1- 9 877 966
- US-B2- 8 710 013

## Description

### CROSS-REFERENCE TO RELATED APPLICATION

This patent application claims the benefit of U.S. Provisional Patent Application No. 62/671,964, filed May 15, 2018.

### STATEMENT REGARDING FEDERALLY SPONSORED RESEARCH OR DEVELOPMENT

This invention was made with Government support under project number 1ZIABC011267-08 by the National Institutes of Health, National Cancer Institute. The Government has certain rights in the invention.

### INCORPORATION-BY-REFERENCE OF MATERIAL SUBMITTED ELECTRONICALLY

Incorporated by reference in its entirety herein is a computer-readable nucleotide/amino acid sequence listing submitted concurrently herewith and identified as follows: one 12,619 Byte ASCII (Text) file named "743502_ST25.txt," dated May 13, 2019.

### BACKGROUND OF THE INVENTION

Metastasis is the cellular mechanism used by disease to spread from an organ to another non-adjacent part of the organism. This process may be involved in the development of solid tumors and may be responsible for the majority of deaths associated with disease. Treatment of a tumoral lesion may have a better prognosis if started in a pre-metastatic stage. In the last decade, although understanding of the underlying mechanisms involved in metastasis has advanced, the therapeutic tools impacting specifically the metastatic process are very limited. Accordingly, there is a need for new formulations and methods of treating subjects suffering from metastatic disease. US9877966B1 provides a pharmaceutical composition for the treatment of a cancer and/or inhibition of metastasis in a mammal that includes a DPYD inhibitor such as gimeracil or a pharmaceutically salt thereof and a compound according to formula I, such as trans-4-(7-benzyl-4-imino-5,6-diphenylpyrrolo[2,3-d]pyrimidin-3-yl)cyclohexan-1-ol, or a pharmaceutically acceptable salt thereof that at least partially inhibits the formation of the perinucleolar compartment and/or inhibits metastasis or migration of cancer cells. The pharmaceutical composition may include at least one excipient. Administration of the pharmaceutical composition may, for example, be oral or parenteral.

### BRIEF SUMMARY OF THE INVENTION

An embodiment of the invention provides pharmaceutical formulations comprising a compound of formula (I): 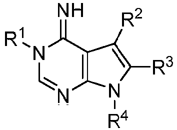 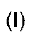 in which R¹, R², R³, and R⁴ are as described herein, or a pharmaceutically acceptable salt thereof, and a pharmaceutically acceptable surfactant comprising one or more caprylocaproyl polyoxylglycerides and PEG-8 caprylic/capric glycerides, further comprising caprylic acid.

A further embodiment of the invention provides the formulations described before for use in treating pancreatic adenocarcinoma in the mammal.

Formulations comprising inhibitors of the perinucleolar compartment ("PNC"), a subnuclear body characterized by its location to the periphery of the nucleolus and which may be associated with malignancy both *in vitro* and *in vivo,* are disclosed as a solution to the unmet need for treating cancer, particularly the metastatic cancers. Compounds of formula (I) are PNC inhibitors. Additionally, methods of treating pancreatic adenocarcinoma by administering a compound of formula (I) are disclosed as a solution to the unmet need for treating pancreatic adenocarcinoma, especially metastatic carcinoma. Further, methods of detecting the change in expression levels of one or both of FoxA1 and FoxO6 in a pancreatic adenocarcinoma tumor sample are disclosed as a solution to the unmet need of determining which mammals will likely respond positively to administration of a compound of formula (I) as a treatment for pancreatic adenocarcinoma.

As seen in the Examples, a compound of formula (I), metarrestin, unexpectedly was selective against metastasis across different preclinical cancer histologies, with high intratumoral exposure and without appreciable toxicity. Metarrestin inhibits Pol I transcription, induces nucleolar segregation, reduces nucleolar volume, and disrupts PNCs, in part by interfering with eEF1A2 function. Further, detecting the change in expression levels of FoxAl and Fox06 in pancreatic adenocarcinoma tumor samples unexpectedly provided valuable insight into which mammals should continue to receive metarrestin.

### BRIEF DESCRIPTION OF THE SEVERAL VIEWS OF THE DRAWINGS

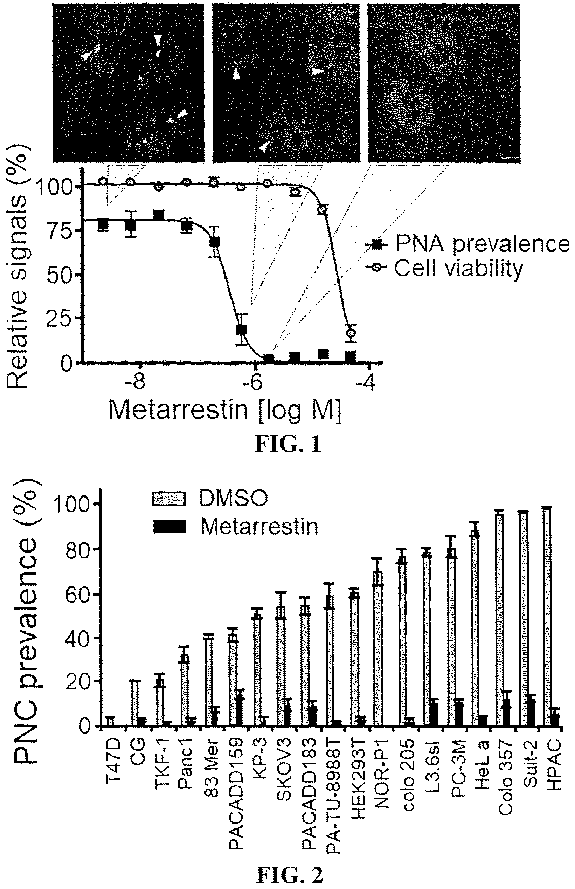

Figure 1 is a graph showing the concentration-response curve against PNC prevalence in PC3M (a metastatic prostate cancer cell line)-GFP (green fluorescent protein)-PTB (polypyrimidine tract-binding protein) cells (squares, PC3M, has a PNC prevalence of 75-85% in the absence of treatment) and cytotoxicity as measured by cellular ATP (CELLTITER GLO^{™}) (circles). Representative PTB-GFP images for cells at the indicated concentrations (arrowheads indicate PNCs) (scale bar = 5 µm) are shown in the three panels above the graph. The relative signal (%) is along the y-axis and Metarrestin [log M] is along the x-axis. As seen from the panels, the amount of fluorescence in the PNC of the cells decreases, with the brightest fluorescence in the left panel and the weakest fluorescence in the right panel. Figures 1-4 show that metarrestin reduces PNCs at a sub-micromolar concentrations and inhibits invasion of cancer cells.
Figure 2 is a bar graph showing that 1 µM metarrestin was effective at reducing PNCs in a range of cancer cell lines (p<0.05 for PNC reduction in all cell lines; the list of cells is in Table 1 below, see also Example 2 below). The DMSO control is shown in light grey bars and metarrestin in dark grey bars. PNC prevalence (%) is on the y-axis and the cell line names are along the x-axis.

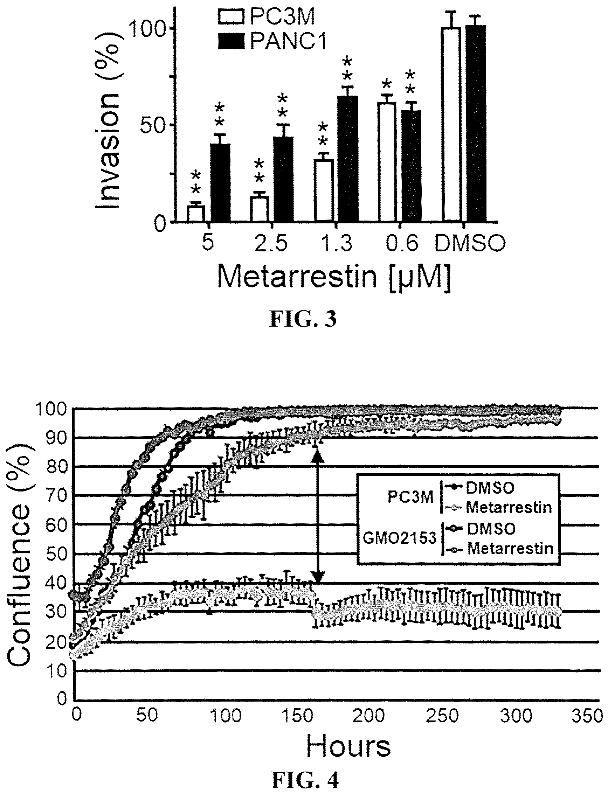

Figure 3 is a bar graph showing that metarrestin inhibits MATRIGEL gelatinous protein mixture (BD Biosciences, Franklin Lakes, NJ) invasion at below micromolar concentrations (0.6 µM) within 24 hours of treatment (* p < 0.05 and ** p < 0.01 in comparison to DMSO [control]). The invasion (%) is on the y-axis and the metarrestin [microM] is on the x-axis. The cell line PC3M is shown in white bars and the cell line PANC1 (human pancreas) is shown in black bars.
Figure 4 is a line graph showing that metarrestin at 1 µM impacts cell growth in PC3M but not in normal fibroblasts (GM02153) (arrow indicates the time of medium change) (* p < 0.05, ** p < 0.01). The confluence (%) is on the y-axis and the time (in hours) is on the x-axis. The top most line is GM02153-DMSO, the second line from the top is PC3M-DMSO, the third line from the top is GM02153-metarrestin, and the bottom line is PC3M-metarrestin.

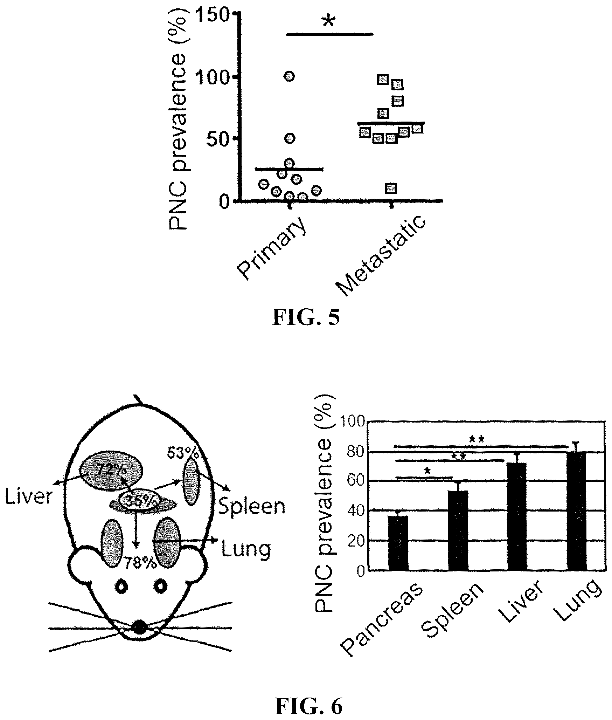

Figure 5 is a graph showing that pancreatic cancer cell lines derived from either primary pancreatic tumors or metastatic lesions showed a higher PNC prevalence in cells derived from metastasis than from primary tumors (cell line explanations in Table 1 below, * p < 0.05). This figure illustrates that metarrestin treatment reduces metastasis to the lungs and liver in NOD/IL2 gamma (null) PANC1 mice. The PNC prevalence (%) is on the y-axis and the type of tumor (primary or metastatic) is on the x-axis.
Figure 6 includes a mouse graphic and bar graph showing PNC prevalence increased in metastatic tissues [shown as circles in the mouse graphic with arrows indicating liver, spleen, and lung] from NOD/IL2 gamma (null) PANC1 mice over primary tumor tissues (35% [center of mouse graphic]), harvested 8 weeks after implantation. PNC prevalence was determined on frozen tissue sections stained with SH54 antibodies. The PNC prevalence (%) is on the y-axis and the type of tumor (i.e., pancreas, spleen, liver, and lung) is on the x-axis (* p < 0.05, ** p < 0.01, *** p < 0.001).

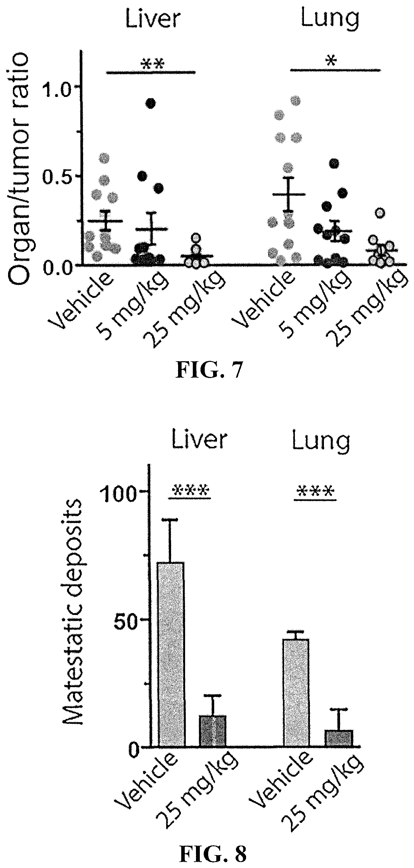

Figure 7 is a graph showing that after six weeks of treatment, metastatic deposits measured by organ/tumor ratio in liver and lungs decreased in mice treated once daily with 25 mg/kg of metarrestin compared to vehicle-treated animals (n=10 mice were randomized to each cohort) (* p < 0.05, ** p < 0.01). The organ to tumor ratio is on the y-axis and the treatment (vehicle, 5 mg/kg, or 25 mg/kg) is on the x-axis for liver (left) and lung (right) tissues.
Figure 8 is a graph showing the results of the pathology examinations which demonstrated that livers and lungs from metarrestin-treated animals have a reduced metastatic burden compared to those treated with vehicle (bar=250 µm; n=4 animals per group analyzed) (*** p < 0.001). The metastatic deposits is on the y-axis and the treatment (vehicle or 25 mg/kg) is on the x-axis for liver (left) and lung (right) tissues.

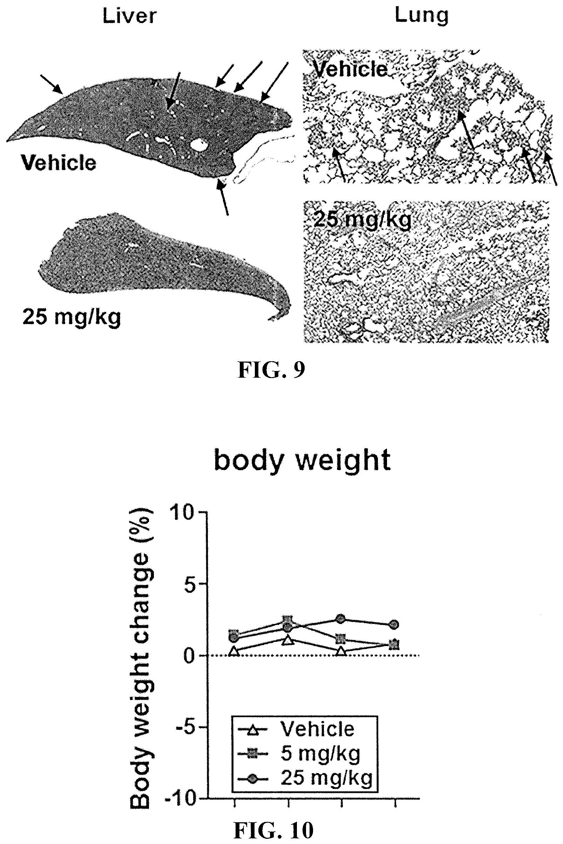

Figure 9 is an illustration showing an example of a histology examination which demonstrated that livers and lungs from metarrestin-treated animals have a reduced metastatic burden compared to those treated with vehicle. Arrows show metastatic tumors.
Figure 10 is a graph showing that the primary tumors in treated animals were not changed following metarrestin treatment. The body weight change is on the y-axis. Vehicle (triangles), 5 mg/kg (squares), and 25 mg/kg (circles) are shown on the graph. The metarrestin treatment was well tolerated, and there were no significant weight differences between treatment groups across the duration of the study.

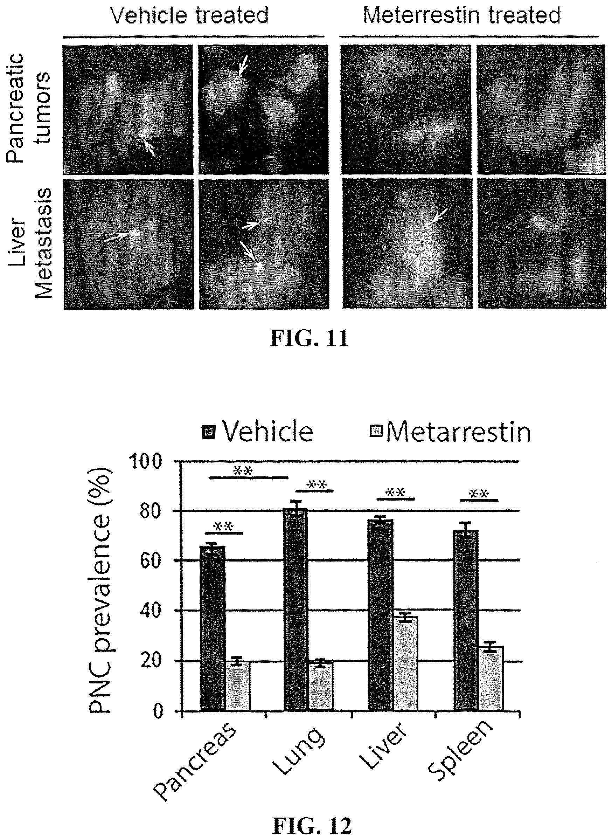

Figure 11 is a set of panels showing that metarrestin disassembles PNCs in primary pancreatic tumors and metastases of NSG PANC1 mice. The PNCs in tumors were visualized via immunofluorescence (PNCs are lightest in color and marked with arrows) 12 weeks after inoculation. Images from the primary tumors and liver metastases are shown (scale bar = 5 µm). Vehicle-treated animals showed typical, easily detectable PNCs. PNC prevalence was reduced and remaining PNCs appeared smaller in metarrestin-treated animals (25 mg/kg IP daily for 6 weeks; n=4 animals per group analyzed).
Figure 12 shows the effect metarrestin has on PNC prevalence in primary pancreatic tumors and sites of metastasis. PNC prevalence (y-axis) was reduced with metarrestin treatment (25 mg/kg IP daily for 6 weeks) in the primary tumor (pancreas) and in metastatic tumors in the lung, liver, and spleen (** p < 0.01). The tumor types (pancreas, lung, liver, and spleen) are along the x-axis.

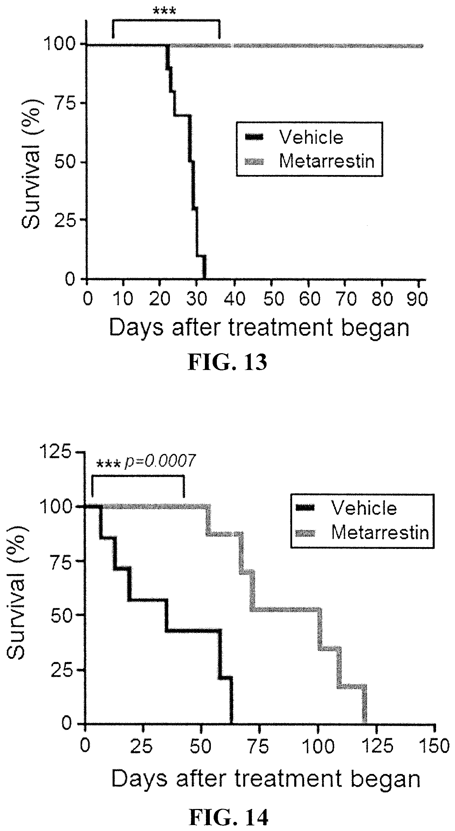

Figure 13 is a graph showing the survival rate of mice following metarrestin or vehicle treatment. This graph illustrates that metarrestin treatment extends survival in the NSG PANC1 pancreatic cancer metastasis model. Metarrestin treatment was provided through drug-infused chow (70 ppm designed to administer 10 mg/kg daily) starting 6 weeks after 3D PANC1 tumor cell inoculation, when animals generally do not show macrometastasis on organ surfaces. Metarrestin treatment prevented mortality beyond 90 days of treatment. The survival rate in the y-axis and the number of days after treatment is on the x-axis. Metarrestin treatment is the solid grey line at 100 % survival and the descending black line is the vehicle (*** p < 0.001).
Figure 14 is a graph showing the survival rate of mice following metarrestin or vehicle treatment. This graph illustrates that metarrestin treatment extends survival in the NSG PANC1 pancreatic cancer metastasis model. Metarrestin treatment was provided through drug-infused chow (70 ppm designed to administer 10 mg/kg daily) after mice developed macrometastasis, including visible liver surface deposits. Metarrestin treatment extended the survival time of the mice compared to vehicle treated (NIH 31 Haslan diet). The survival rate in the y-axis and the number of days after treatment is on the x-axis. Metarrestin treatment is the solid grey line and the black line is the vehicle (*** p = 0.007).

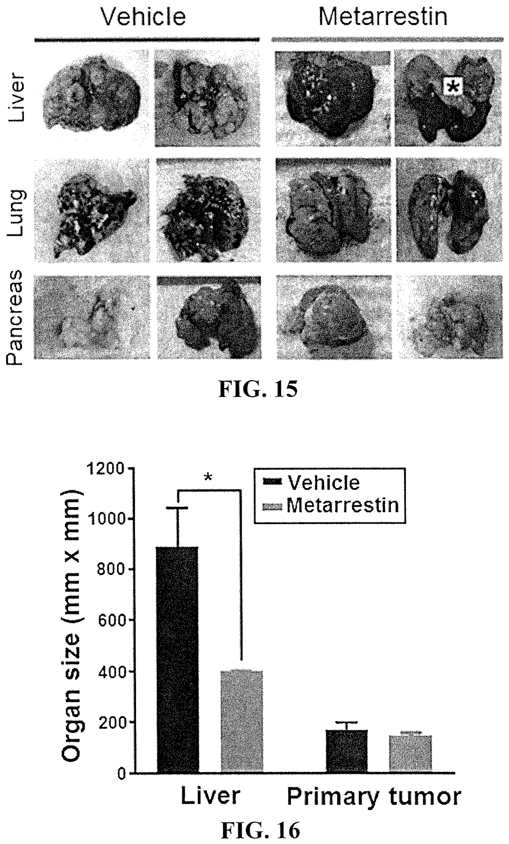

Figure 15 is a series of panels which show exemplary tissues following full necropsy of mice at the time of death. These panels demonstrate decreased metastatic disease burden in the liver of metarrestin-treated animals without detectable impact on primary tumor size. Animals in the control group showed near complete or complete organ replacement with tumors, particularly in the liver (*indicates thick right hemidiaphragm) and to a lesser degree in the lung. Pancreatic tumors, in comparison, were similar in both groups. Tissues from the vehicle treated animals are in the 2 left columns and tissues from the metarrestin treated animals are in the right 2 columns. Liver tissues are shown in the top row, lung tissues are shown in the middle row, and pancreas tissues are shown in the bottom row.
Figure 16 is a graph showing the average sizes of livers (containing metastatic tumors) and of primary pancreatic tumors in vehicle and metarrestin-treated mice from the study in Fig. 14. (* p < 0.05). Organ size (mm x mm) is on the y-axis and tumor type is on the y-axis (metastatic liver and primary pancreas). Vehicle is represent by the black bars and metarrestin represented by the grey bars.

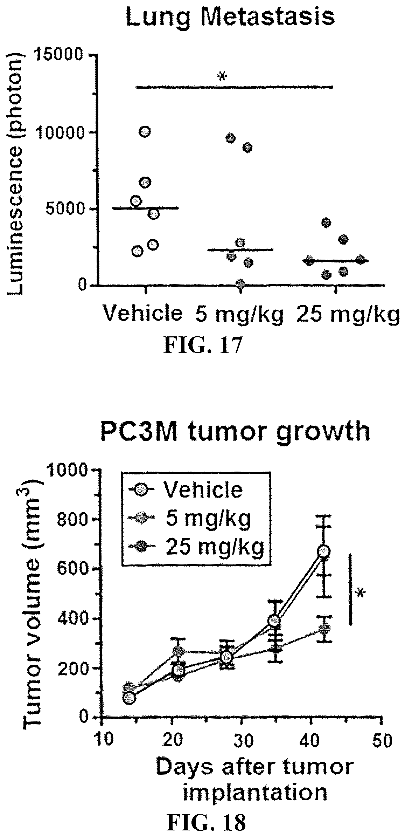

Figure 17 is graph showing that daily treatment with metarrestin significantly reduced lung metastasis as measured by quantitative in vivo imaging systems ("IVIS") (n=6 for each group). The luminescence (photon) is on the y-axis and the treatment (i.e., vehicle, 5 mg/kg, and 25 mg/kg metarrestin) is on the y-axis (* p < 0.05).
Figure 18 is a graph showing that metarrestin treatment had a small effect on the growth of PC3M primary tumors inoculated SC, as measured by tumor volume (y-axis, in mm³). The number of days after tumor implantation is along the x-axis (* p < 0.05).

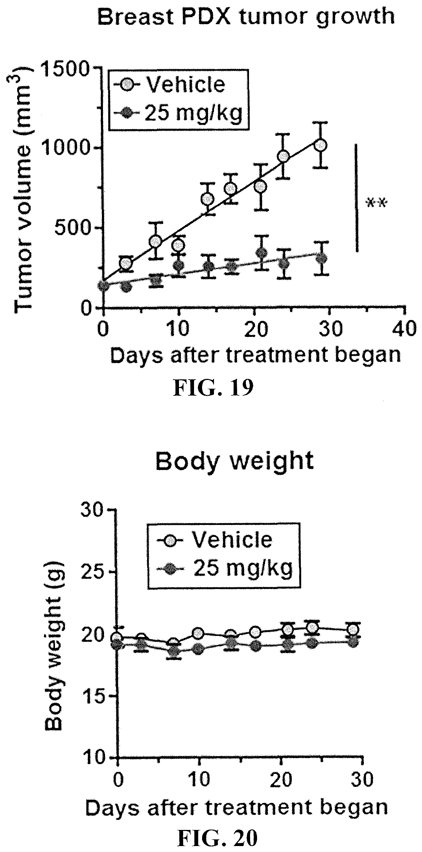

Figure 19 shows that metarrestin treatment effectively inhibited the growth of a PDX consisting of metastatic cells from a patient's pleural fluid (n=5 for each group), as measured by tumor volume (y-axis, in mm³) and tumor weight at the end of the experiment. The number of days after treatment began is along the y-axis (** p < 0.01).
Figure 20 is a graph showing that the weekly body weight evaluations did not show significant differences between treated and control groups. The body weight of treated animals was not changed. Animals treated with metarrestin remained agile and well-groomed, in contrast to vehicle-treated animals.

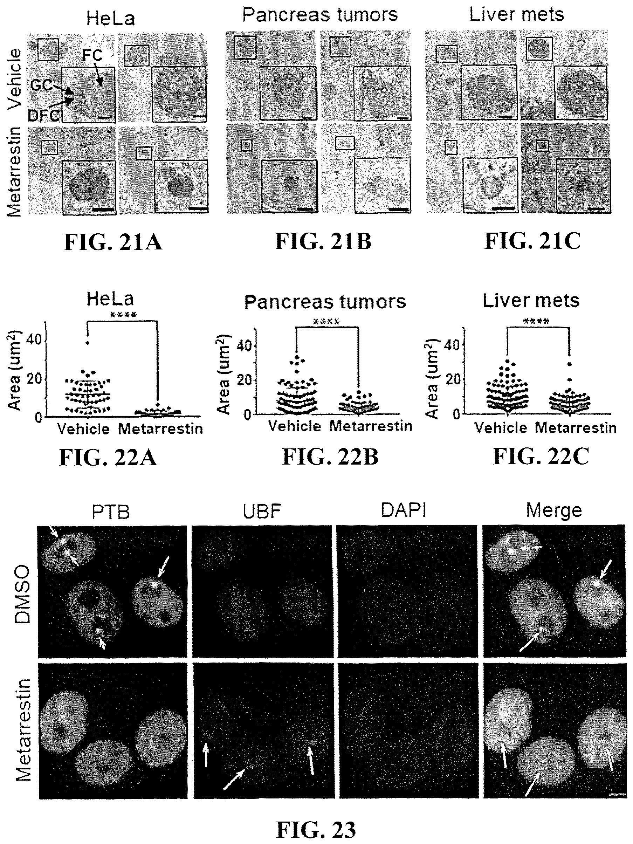

Figures 21A-21C are a set of panels showing that nucleoli lose their typical three substructures, as seen in untreated or DMSO-treated cells (arrows indicate DFC, dense fibrillary components, FC, fibrillar components, and GC, granular components), and develop nucleolar capping (enlarged inserts) upon treatment with metarrestin in HeLa cells and tumor tissues. Representative electron micrographs are shown for HeLa cells (Figure 21A; treated at 1 µM for 24 hours), primary pancreatic tumors (Figure 21B), and liver metastases (Figure 21C) from NSG PANC1 mice treated with vehicle (top) or 10 mg/kg metarrestin (bottom) for 7 days. Mice were harvested 1 hour after the last metarrestin dose (inset shows nucleoli). Scale bar = 1 µm. Figures 21A-24 show that metarrestin treatment induces nucleolar structure changes.
Figures 22A-22C are three graphs showing quantitative evaluation of the EM images which demonstrated that the average nucleolar area was reduced in metarrestin-treated cell lines and tissues compared to vehicle control (P < 0.0001) in HeLa cells (Figure 22A), primary pancreatic tumors (Figure 22B), and liver metastases (Figure 22C). 100 nucleoli were randomly selected and analyzed to calculate nucleolar area as ((largest+shortest diameter)/2)²×π). The comparison of mean nucleolar areas was performed using Mann Whitney U test, 2-tailed, n=4 animals per group.
Figure 23 is a series of panels showing the changes in nucleolar architecture induced by metarrestin treatment which are reflected in the redistribution of Pol I transcription factor, UBF, into cap-like structures (capping) (arrows), corresponding to the loss of PNCs (top left panel). As shown in the merge panel, the capping of UBF reflects the segregation of the fibrillar components from the granular components, as seen in EM images in Fig. 21A-21C. Scale bars = 5 µm.

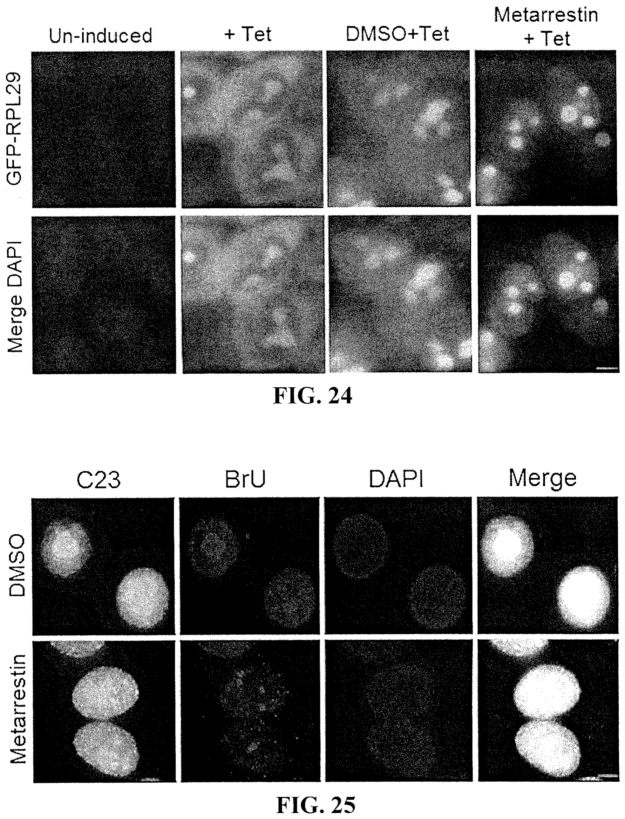

Figure 24 is a panel showing that metarrestin interferes with ribosomal biogenesis. An inducible RPL29-GFP-expressing cell line synthesizes RPL29-GFP when treated with tetracyclin (2^{nd} column). When cells were treated with 1 µM metarrestin before tetracycline induction, the newly synthesized proteins accumulated in the nucleoli and nuclei (right most panels) compared to the DMSO control treated cells (2^{nd} from right panels). Scale bars = 5 µm.
Figure 25 is a set of panels showing that Pol I transcription patterns are altered, as demonstrated by a redistribution of BrU incorporation signals from typical nucleolar labeling to pin-points at 5 min (2^{nd} from left column), corresponding to the changes in nucleolar structure shown as a loss of nucleolar labeling of C23/nucleolin (left column). All cells treated with metarrestin showed alterations of BrU labeling in the nucleoli compared to a small fraction of cells in DMSO treated cells (right panel). Figures 25-34 show that metarrestin treatment reduces pre-RNA synthesis and Pol I occupancy at rDNA without changing rDNA chromatin states.

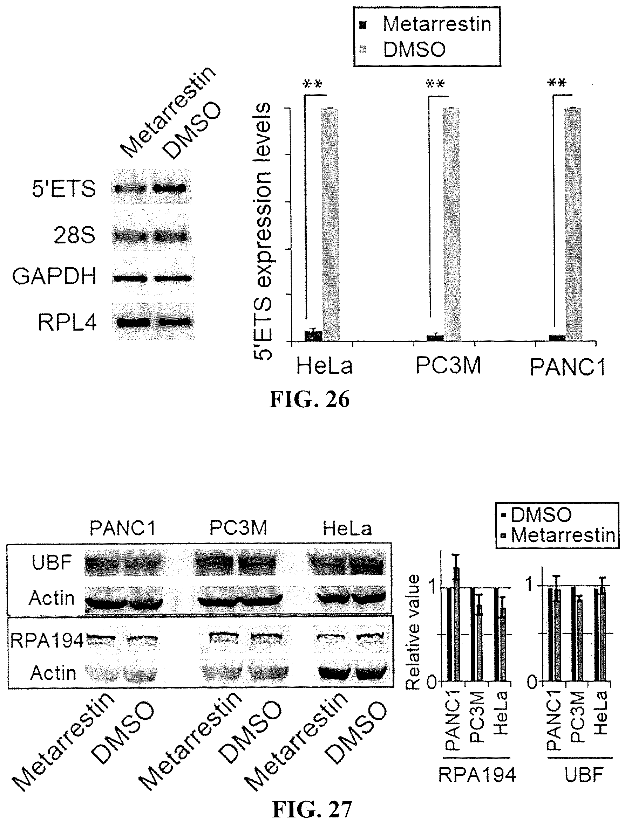

Figure 26 shows the results of RT-PCR (left panel) and qRT-PCR (right panel) which illustrate the reduction in 5'ETS of the pre-rRNA in metarrestin-treated cells.
Figure 27 shows Western blot results which indicate that no changes in protein expression of RPA194, the large subunit of Pol I, and UBF are present in metarrestin-treated cells.

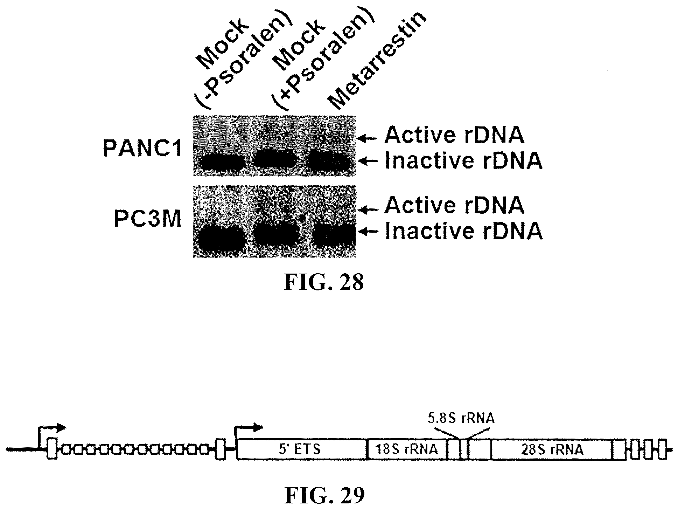

Figure 28 shows the results of psoralen-crosslinking experiments illustrating that the ratio of active to inactive rDNA chromatin appears unchanged upon exposure to metarrestin.
Figure 29 is a diagram showing the rDNA structure.

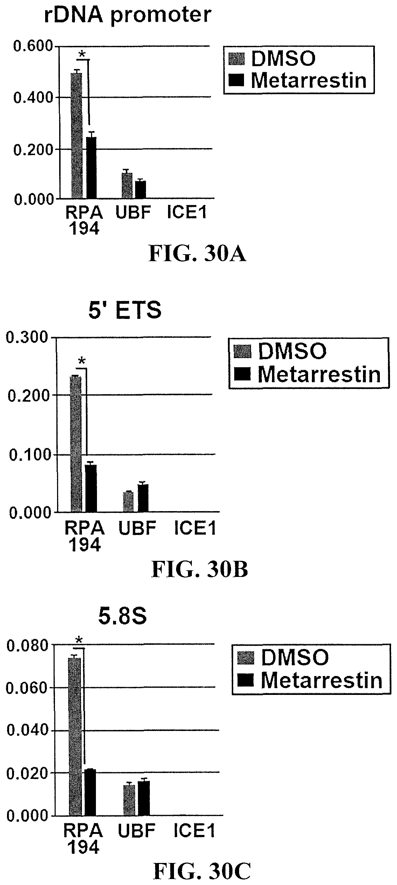

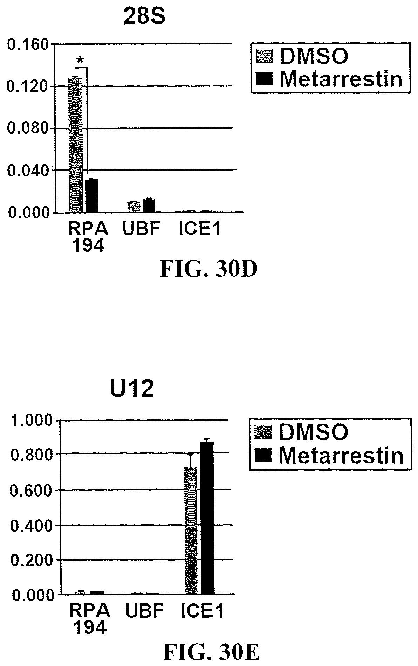

Figures 30A-30E are a set of graphs showing quantitative ChIP evaluations which illustrate that metarrestin treatment reduces the occupancy of RPA194, but not UBF, on rDNA through the promoter and the coding region (Figure 30A is rDNA promoter, Figure 30B is 5' ETS, Figure 30C is 5.8S, Figure 30D is 28S, and Figure 30E is U12).

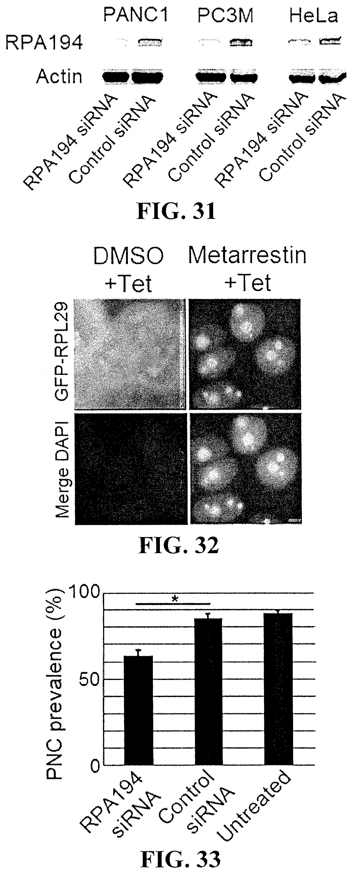

Figure 31 shows that knockdown of pol I by siRNA showed reduction of RPA194 as measured by Western blots, and the amount was quantified in relation to control siRNA treated cells (set as 1).
Figure 32 is set of panels showing that knockdown of RPA194 by siRNA reduced PNC prevalence and increased the number of PNCs with a crescent shape (grey portion).
Figure 33 is a graph showing PNC prevalence. The PNC prevalence (%) is on the y-axis and the type of cell is on the x-axis (* p < 0.05).

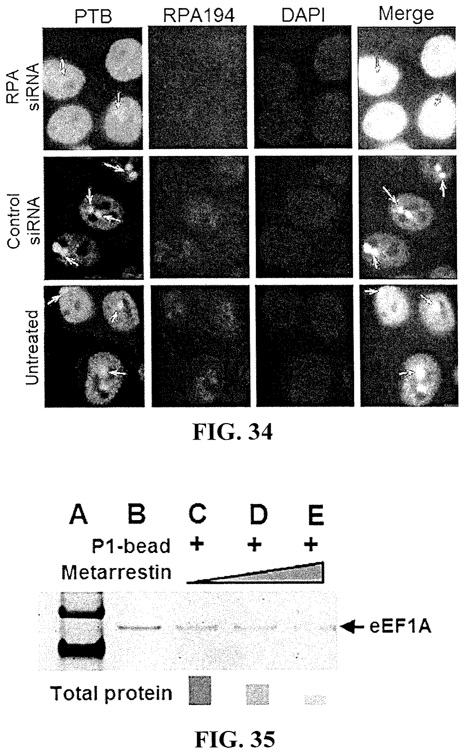

Figure 34 is a set of panels showing RPA194 knockdown also disrupts the nucleolus (top row, 2^{nd} from left column, white arrows) compared to untreated and control oligo treated cells (lower two panels). PNC structures were altered into crescent shapes (top row, left column, arrows) compared to untreated or control oligo treated cells (lower two rows) (* p<0.05, ** p<0.01). Scale bars for all images = 5 µm.
Figure 35 is a Western blot illustrating that metarrestin effectively outcompeted recombinant eEF1A from binding to anchored biotinylated metarrestin-eEF1A complex. Fig. 35-40C show that metarrestin specifically binds eEF1A2. Increased eEF1A2 enhances PNCs and metastasis formation.

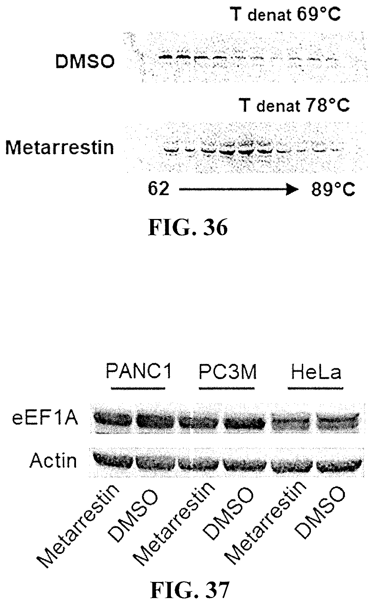

Figure 36 is a gel that shows that metarrestin treatment stabilized eEF1A in a thermal stability assay using PC3M cell lysate. DMSO is the top panel and metarrestin is the bottom panel.
Figure 37 shows a Western blot analyses which indicates that there were no changes in the amount of eEF1A proteins upon metarrestin treatment at 1 µM for 24 hours.

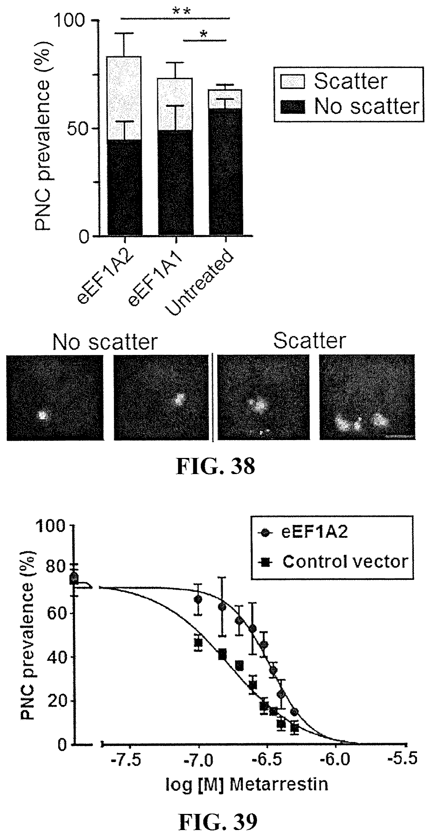

Figure 38 is a graph and set of panels. The top shows that overexpression of HA-eEF1A2 increased the number of PNCs per nucleus (scattered PNC prevalence: the number of cells containing 2 or more PNCs); n = 300 cells (bar= 2 µm). The graph shows that it did not significantly increase overall PNC prevalence. Scatter is represented by the light grey bars and no scatter is represented by black bars. PNC prevalence (%) is on the y-axis and eEF1A2, eEF1A1, and untreated are along the x-axis (* p < 0.05, ** p < 0.01).
Figure 39 is a graph showing that overexpression of eEF1A2 in PC3M cells increased the IC₅₀ of metarrestin for PNC disassembly. PNC prevalence (%) is on the y-axis and the log [M] metarrestin is on the y-axis. eEF1A2 is shown by circles and the control vector is shown by squares.

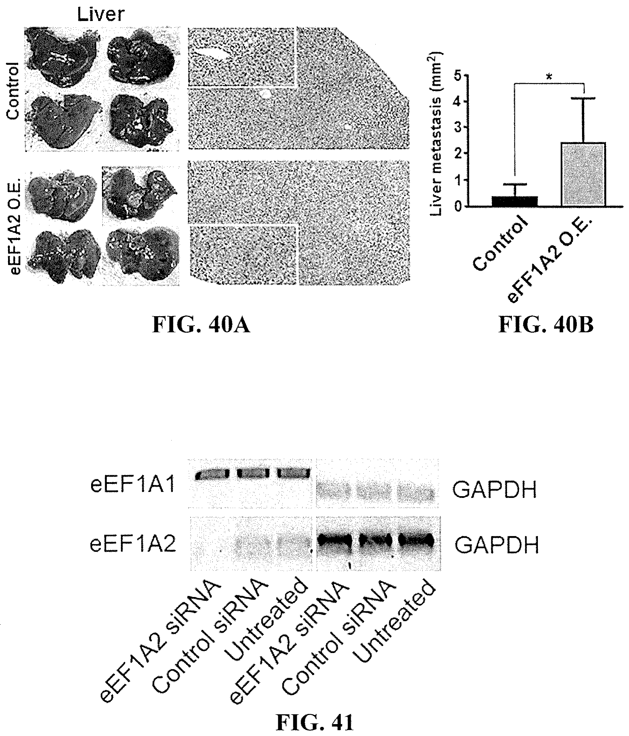

Figures 40A-40B show the results when 6×10⁴ PANC1 3D spheres transduced with empty vector (control) or eEF1A2 (eEF1A2 O.E.) were injected into the tail of the pancreas of NSG mice. Mice from both groups were harvested 6 weeks after implantation and subjected to necropsy. Figure 40A shows the macroscopic images of anterior (top panel) and posterior (bottom panel) liver surfaces showed higher metastatic burden in PANC eEF1A2 animals than in empty vector control (left, harvested livers). Histopathological images (H&E stain) of livers (black scale bar = 250 µm, white scale bar = 100 µm) are shown to the right of the macroscopic images. Insets depict representative metastatic lesions. Figure 40B shows the quantification of the liver metastasis and shows that the livers metastasis had a higher metastatic burden in PANC1 eEF1A2 O.E. animals (n=4 animals analyzed per group) (* p < 0.05). The number of liver metastasis (mm3) is shown on the y-axis and the control and eEF1A2 O.E are on the x-axis.
Figure 41 is a gel showing that seventy-two hours after siRNA transfection into HeLa cells, eEF1A2 RNA, but not eEF1A1 RNA, was reduced, as measured by RT-PCR. Fig. 41-45B show that eEF1A2 reduction induces similar nucleolar and PNC disruption as metarrestin.

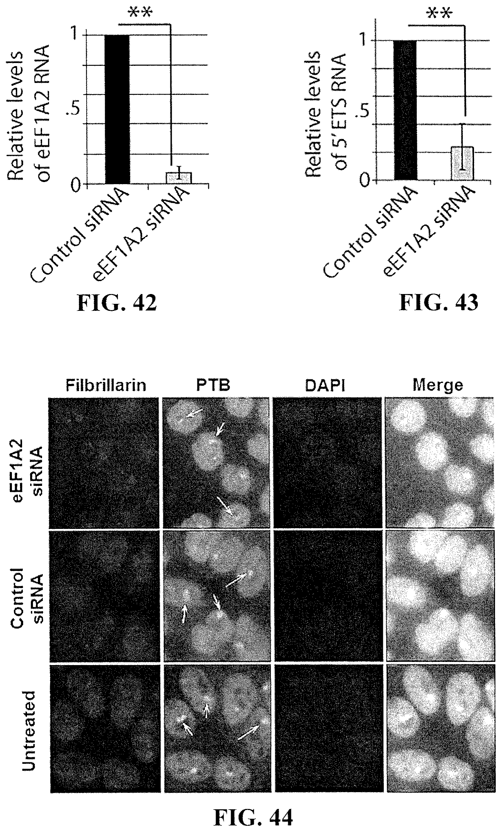

Figure 42 is a graph showing that qRT-PCR showed a reduction of eEF1A2 RNA in siRNA-transfected cells. The relative levels of eEF1A2 RNA is on the y-axis (** p < 0.01).
Figure 43 is a graph showing that seventy-two hours after transfection with eEF1A2 siRNA, the amount of 5'ETS RNA was reduced, as measured by qRT-PCR.
Figure 44 is a panel showing that nucleolar and PNC disruption was detected using immunofluorescence in siRNA transfected cells. The PNCs in eEF1A2 knock-down cells generally showed crescent shapes (arrows), as well as segregated nucleoli (capping, arrows) immunolabeled with an antibody recognizing a pre-RNA processing factor fibrillarin; n = 500 cells. Scale bar = 5 µm.

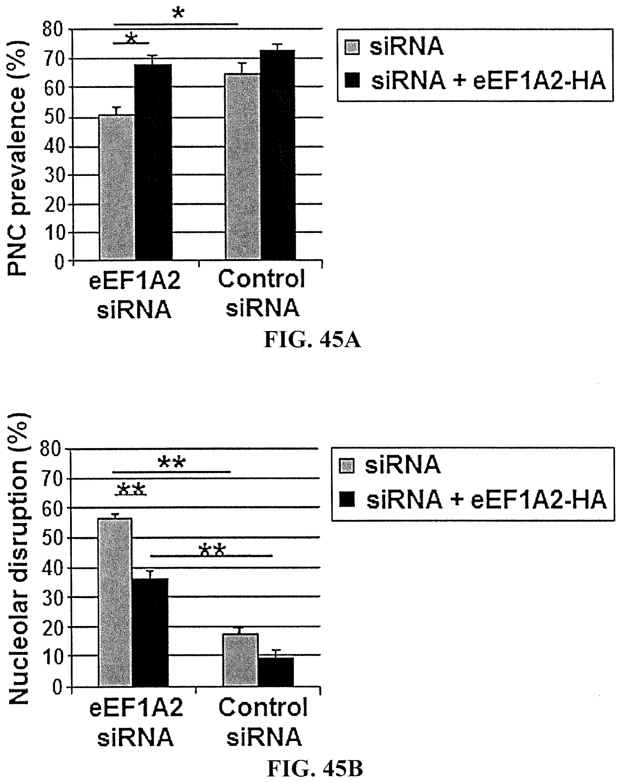

Figures 45A-45B are graphs showing that PNC prevalence was modestly reduced (Figure 45A), and the rate of nucleolar disruption was increased (Figure 45B). Transfection of HA-eEF1A2 (black bars) after siRNA for additional 24 hours partially rescued PNC prevalence (Figure 45A, black bars) and nucleolar disruption (Figure 45B, grey bars) (* p < 0.05, ** p < 0.01).

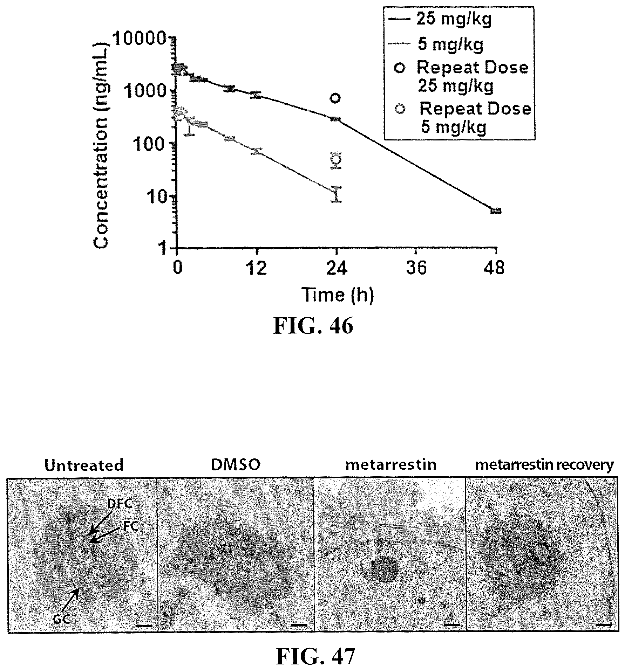

Figure 46 shows plasma pharmacokinetics ("PK") of metarrestin when administered as a single dose, intraperitoneal injection. Metarrestin was administered to female BALB/c mice, n=3 in the single dose and the repeat doses were administered to SCID-beige mice, n=3 per time point. The repeat dose study mice were given daily intraperitoneal injections for 4 weeks, with concentration measured 24 h after the last dose. Metarrestin was formulated in 5% NMP + 20% PEG400 + 75% (10% HP-β-CD in water).
Figure 47 is a set of panels which show metarrestin-induced nucleolar structure changes as observed by electron microscopy. The structure of the nucleolus loses the typical three substructures seen in untreated or DMSO-treated cells (DFC, dense fibrillary components, FC, fibrillar components,and GC, granular components, are specified by arrows and labels), and becomes highly segregated (metarrestin panel) upon treatment with metarrestin at 1 µM for 24 hours in HeLa cells. However, withdrawing the treatment allows for the recovery of the nucleolus (metarrestin recovery panel). Scale bar = 500 nm.

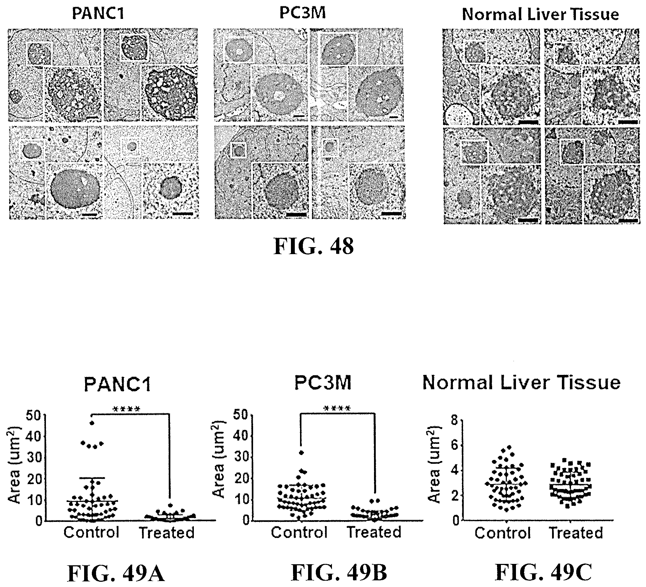

Figure 48 is a set of panels showing that similar changes are observed in PANC 1 and PC3M cells (left two panels), but not observed in treated normal liver tissues (right panels). Scale bar = 1 µm.
Figures 49A-49C are graphs showing quantitative evaluation of the EM images of Figure 48. Figure 49 demonstrated that nucleolar volume was reduced in the treated cancer cell lines (Figures 49A-49B), but not in treated normal liver tissues (Figure 49C) (**** p <0.0001).

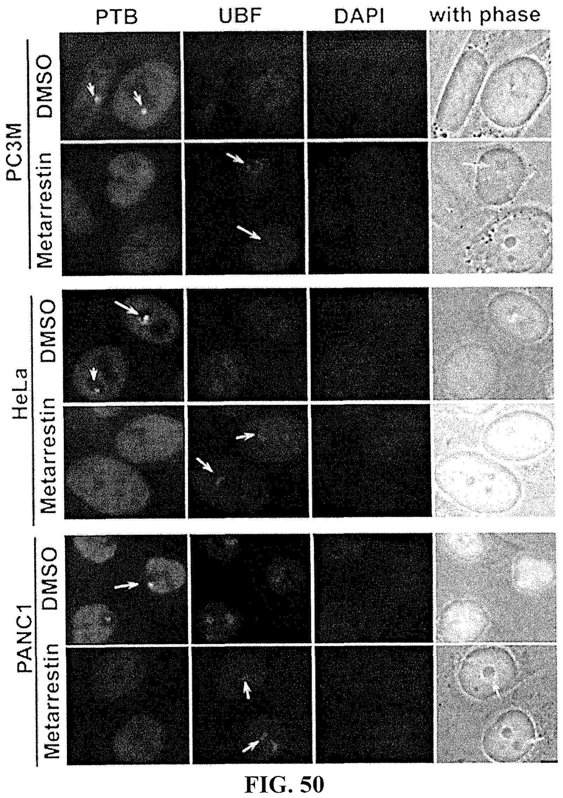

Figure 50 is a set of panels showing changes in nucleolar structure in metarrestin-treated cells. The nucleolar structure changes (UBF labeling in arrows) are closely associated with the loss of PNC. Metarrestin treatment (1 µM for 18 hours) was tested in three cell lines, PANC1, PC3M, and HeLa. The capping of the Pol I transcription factor UBF in the metarrestin-treated cells is evident as shown in merged images (indicated by arrows). Scale bar = 10 µm.

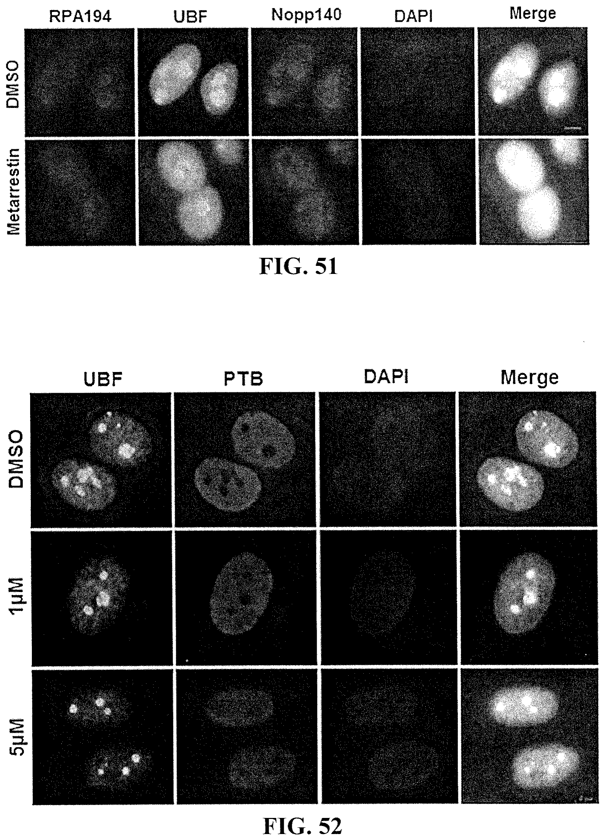

Figure 51 is a set of panels showing the capping structure of Pol I transcription factors in metarrestin-treated cells. Pol I transcription factors, UBF (column 2^{nd} from left), RPA194 (left column), and pre-ribosomal RNA processing factor, NOPP140 (middle column) showed similar capping structure in metarrestin-treated cells. Scale bar = 10 µm.
Figure 52 is a set of panels showing the immunolabeling of two pre-ribosomal RNA processing factors, fibrillarin and NOPP140, demonstrated the cap-like structure in metarrestin-treated cells (arrows). Scale bar = 5 µm.

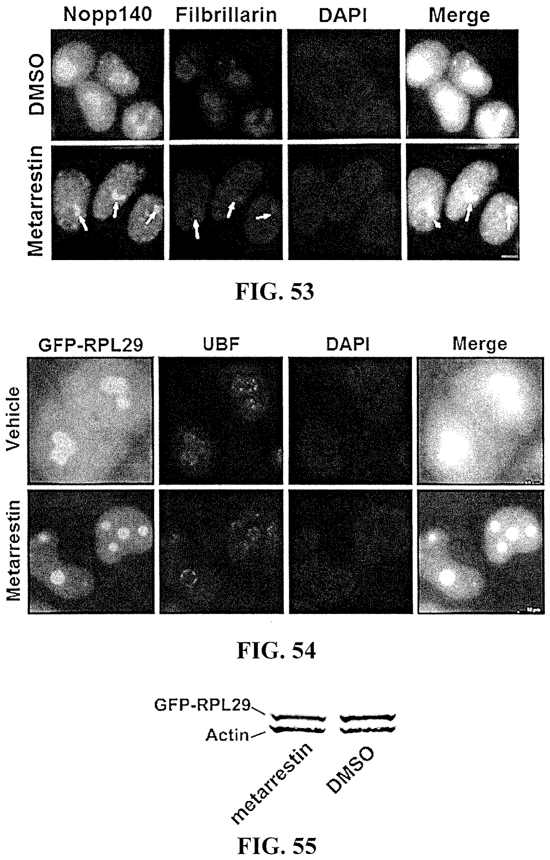

Figure 53 is a set of panels showing that the capping of Pol I transcription factors was not evident in normal fibroblast cell line, GM02153, under metarrestin treatment with the same protocol (1 µM for 18 hours). These cells do not have PNCs as PTB labeling did not show any perinucleolar enriched labeling. UBF did not become completely capped even when treated with 5 µM in these cells. Scale bar = 5 µm.
Figure 54 is a set of panels showing that in metarrestin treated cells, nucleoli altered with nucleolar capping can be detected by immunolabeling of UBF. The newly synthesized GFP-RPL29 entered nuclei and localized to the distorted nucleoli, but was not assembled into mature ribosomes in metarrestin-treated cells to be transported into the cytoplasm. Scale bar = 10 µm.
Figure 55 is a gel showing that GFP-RPL29 expression was not significantly changed when stably transfected HeLa cells were induced to express the GFP-RPL29 fusion protein after treatment with metarrestin (1 µM for 5 hours). Figures 54 and 55 together show that there is no impact of metarrestin treatment on Pol II transcription, translation, or cytoplasmic-nuclear trafficking in metarrestin-treated cells.

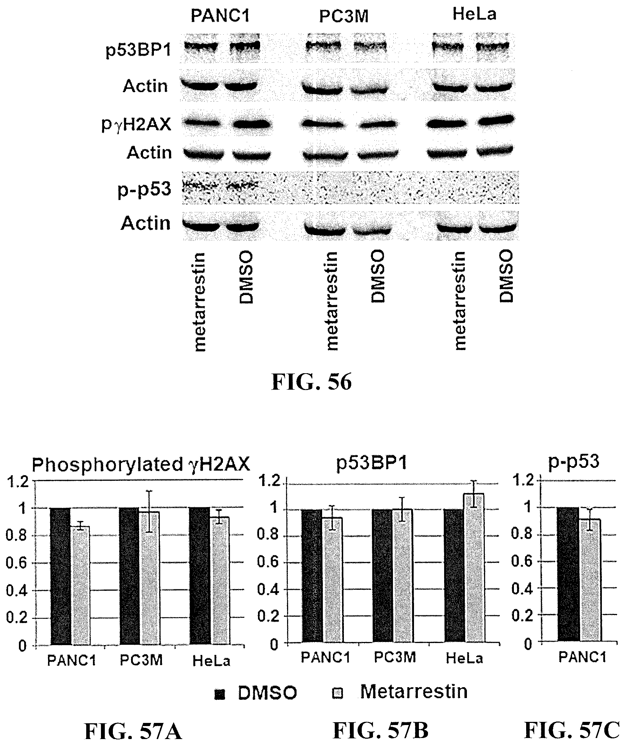

Figure 56 is a Western blot showing that there was no impact on DNA damage response, cell cycle, or pol II transcription in metarrestin-treated cells. Specifically, the Western blot analyses of DNA damage response signature phosphorylated proteins or p53.
Figures 57A-57C are graphs showing that there were not significant changes when cells were treated with metarrestin at 1 µM for 24 hours. Figure 57A is phosphorylated yH2AX, Figure 57B is p53BP1, and Figure 57C is p-p53. The cell lines are along the x-axis and the amounts of change are on the y-axis. DMSO is represented by black bars and metarrestin is represented by grey bars.

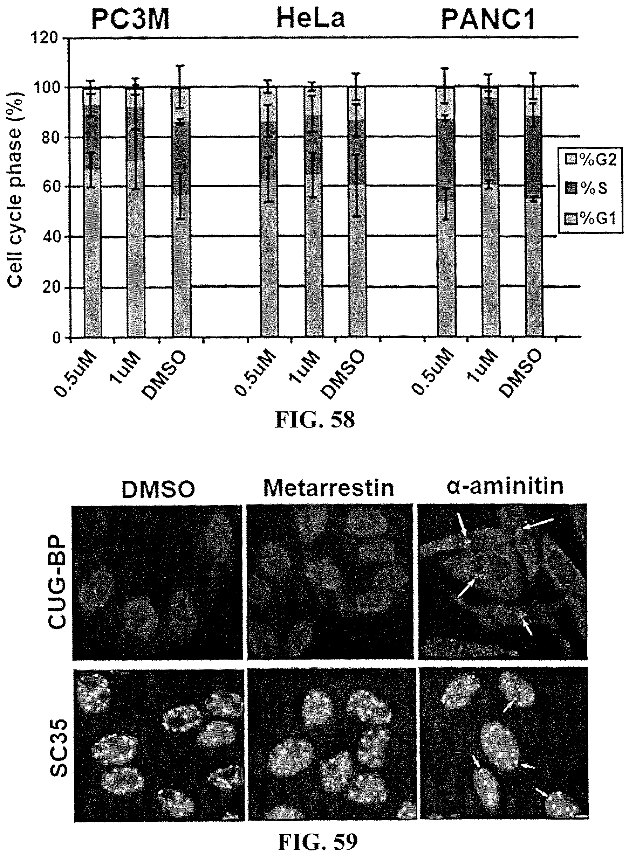

Figure 58 is a graph showing the cell cycle analyses of DNA content by flow cytometry. The study indicates that there was not significant cell cycle block within 24 hours of metarrestin treatment at two different concentrations. The cell cycle phase (%) is on the y-axis.
Figure 59 is a set of panels showing that the immunolabeling of CUGBP and SC35 in metarrestin-treated cells did not show the signature changes of their labeling patterns that are seen during pol II transcription inhibition by α-amanitin. Metarrestin treatment disassembles PNCs without causing cytoplasmic relocation of CUGBP (top middle panel), as compared to treatment by α-amanitin, which induces cytoplasmic relocation of the protein, but does not affect proteins localized to the PNCs (top right panel, arrows). Similarly, metarrestin treatment did not significantly impact the speckled distribution pattern (bottom middle panel), unlike α-amanitin that induced round aggregates (bottom right panel, arrows). Scale bar = 5 µm.

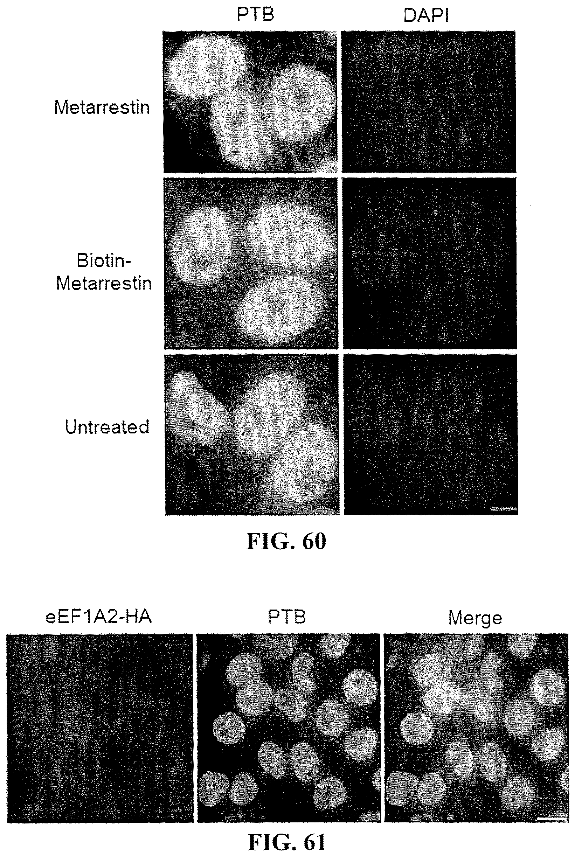

Figure 60 is a set of panels showing the effectiveness of biotin-metarrestin in disassembling PNCs. The effects of metarrestin and biotin-metarrestin were compared in PC3M cells treated at 1 µM or untreated for 24 hours. Arrows indicate PNCs. Scale bar= 5 µm.
Figure 61 shows expression of eEF 1A2-HA in transfected cells. The protein is predominantly in the cytoplasm. Immunolabeling using SH54 demonstrates the nucleoplasmic and PNC distribution of PTB. The transfection efficiency was around 70%. (*** p < 0.001). Scale bar = 20 µm.

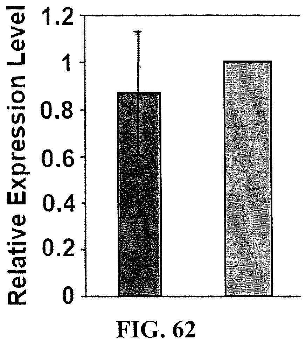

Figure 62 is a graph showing that there was no impact of metarrestin treatment on Pol II transcription, translation, or cytoplasmic-nuclear trafficking in metarrestin-treated cells. Specifically, stably transfected HeLa cells were induced to express the GFP-RPL29 fusion protein after treatment with metarrestin (1µM for 5 hours). GFP-RPL29 expression was not significantly changed.

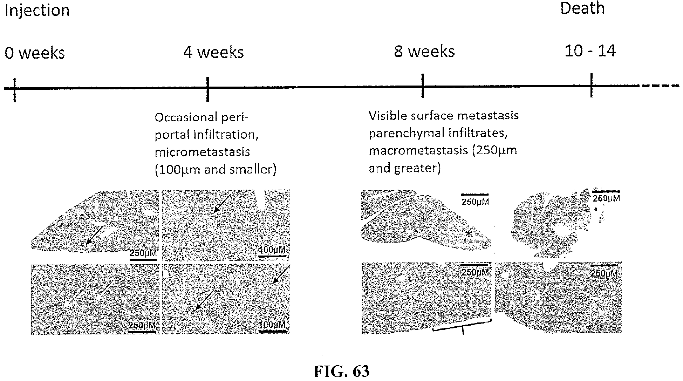

Figure 63 is a graph showing the drug response of PANC1 cells following metarrestin treatment. Relative cell growth was measured by CELLTITERGLO^{™} normalized to DMSO control samples (set to 1.0) after 72 hours. Mean cell viability values are plotted with standard error of the mean (SEM) from at least 2 independent experiments done in triplicate.

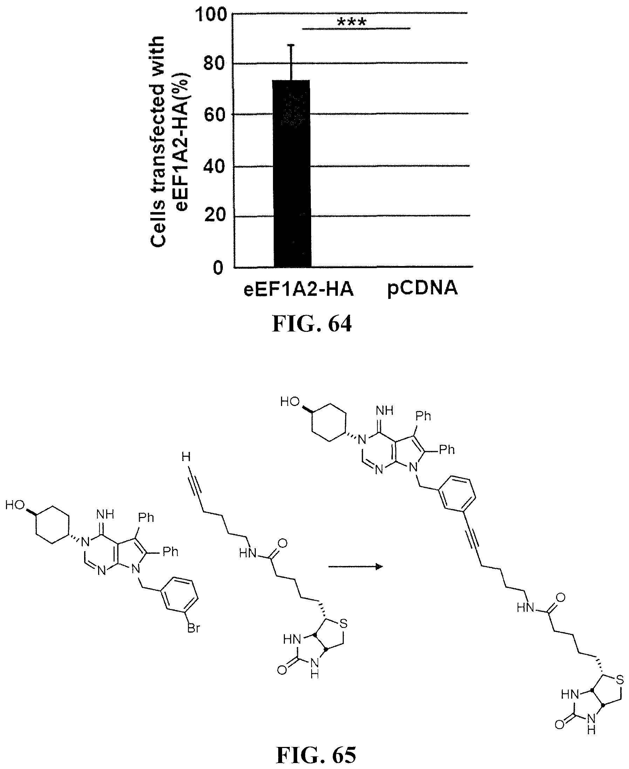

Figure 64 shows that metastatic cancer progression in PANC1 NSG mice. After intrapancreatic injection of sixty thousand luciferase-expressing 3D PANC1-luc cells derived from PANC-luc cells grown as spheroids in NSG mice, micrometastasis (black arrows) with periportal infiltration (white arrows) developed within approximately 4 weeks (representative H&E staining, - scale bar=250 µm, white scale bar=100 µm). Macrometastasis with visible surface metastases (indicated with -) developed after 8 weeks. * indicates necrosis. Timeline depicted at the top.
Figure 65 shows the synthesis of N-(6-(3-((3-(trans-4-hydroxycyclohexyl)-4-imino-5,6-diphenyl-3,4-dihydro-7H-pyrrolo[2,3-d]pyrimidin-7-yl)methyl)phenyl)hex-5-yn-1-yl)-5-((3aS,4S,6aR)-2-oxohexahydro-1H-thieno[3,4-d]imidazol-4-yl)pentanamide (biotin-metarrestin). Specifically, a modified Voigt reaction/Knoevenagel condensation sequence was carried out using the procedure of Roth and Eger (Synthesis of 2-amino-3-cyano-pyrroles (author's transl), ArchPharm (Weinheim), 308: 179-85 (1975)). Thus, benzoin (5.26 g, 24.8 mmol), 3-bromobenzylamine (4.61 g, 24.8 mmol), and trifluoroacetic acid (0.14 g, 1.24 mmol, 0.05 equiv.) were heated at reflux for 1 h connected to a Dean-Stark trap, and then the mixture was removed from the oil bath. Malononitrile (4.91 g, 74.3 mmol, 3.0 equiv.) was added to the mixture, and the reaction again heated at reflux for 1 h connected to a Dean-Stark trap. The reaction mixture was allowed to cool to room temperature and stirred for 19 h, affording the crude pyrrole as a dark red solid. The product was further precipitated with ethyl ether and the solid washed with additional ethyl ether until the filtrate was colorless, affording the pyrrole product as a light purple solid (6.80 g, 15.87 mmol, 64% yield), which was used without further purification. R*f* = 0.53 (50% EtOAc/hexanes); mp = 184-190 °C; 1H NMR (400 MHz, DMSO-d6) δ 4.96 (s, 2 H), 6.80 (d, *J=* 7.8 Hz, 1 H), 7.03-7.28 (complex, 12 H), 7.43 (d, *J=* 9.0 Hz, 1 H); 13C NMR (101 MHz, DMSO-d6, APT pulse sequence) δ d (CH, CH3): 125.6, 126.6, 128.3, 128.5, 128.9, 129.0, 129.6, 130.4, 131.1, 131.5; u: (C, CH2): 45.4, 118.4, 120.7, 122.0, 124.0, 131.3, 133.9, 140.4, 149.1; IR 2203, 1632 cm-1; HRMS (ESI) m/z calculated for C24H19BrN3 [M + H]+ 428.0757, found 428.0749.

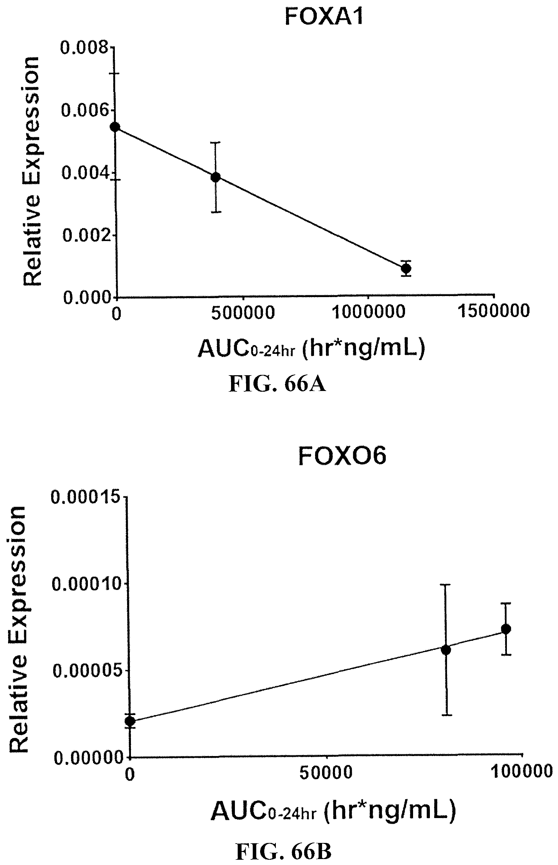

Figures 66A-66B are graphs showing the relative expression of biomarkers FoxA1 (Figure 66A) and FoxO6 (Figure 66B). The relative expression is on the y-axis and the AUC_{0-24 hr} (hr*ng/mL) for metarrestin is on the x-axis in both graphs (R₂ = 0.9999; p = 0.0076 for FoxAl and R₂ = 0.9952; p = 0.0440 for FoxO6).

### DETAILED DESCRIPTION OF THE INVENTION

### Formulations

An embodiment of the invention provides formulations comprising a compound of formula (I), 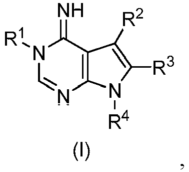 wherein
R₂ is phenyl, R³ is phenyl, R⁴ is benzyl, and R¹ can be any of the following structures:

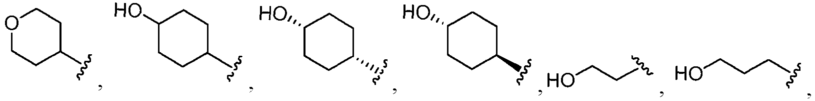

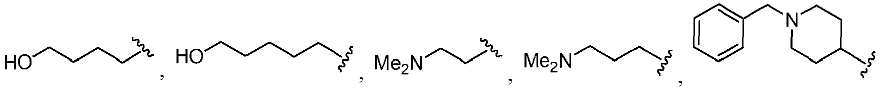

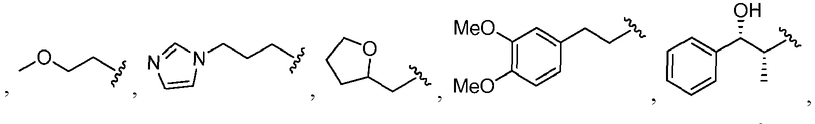

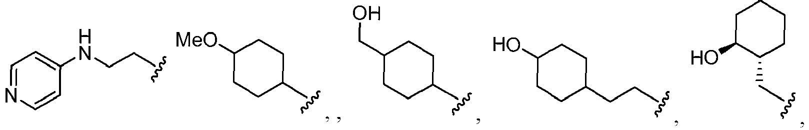

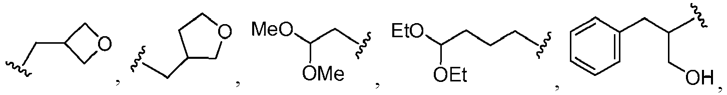

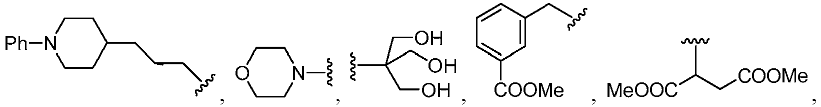

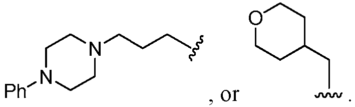

In an embodiment, R² is phenyl, R³ is phenyl, R⁴ is benzyl, and R¹ can be any of the following structures: 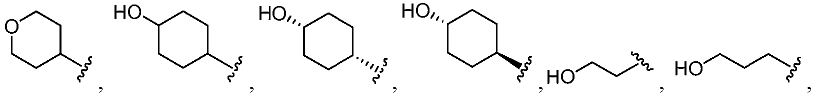 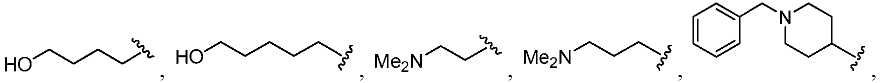 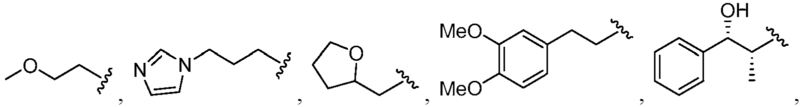 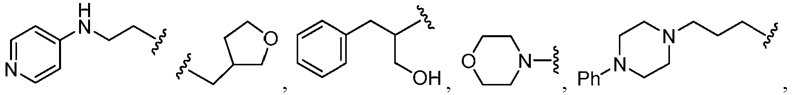 or

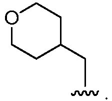

In an embodiment, R² is phenyl, R³ is phenyl, R⁴ is benzyl, and R¹ is

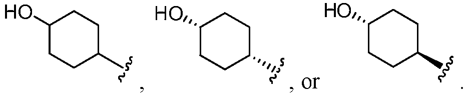

In an embodiment, the compound of formula (I) is metarrestin (see U.S. Patent No. 9,663,521).

An embodiment of the invention provides a formulation comprising a pharmaceutically acceptable surfactant comprising one or more caprylocaproyl polyoxylglycerides and one or more PEG-8 caprylic/capric glycerides. In an embodiment, the formulation is administered by any one of the following methods: oral, aerosol, nasal, pulmonary, parenteral (e.g., intravenously ["IV"], subcutaneously, intradermally, or intramuscularly), subcutaneous, intravenous, intramuscular, intraperitoneal, intrathecal, intratumoral, topical, rectal, and vaginal. Preferably, the formulation is suitable for oral administration.

A suitable source of one or more caprylocaproyl polyoxylglycerides and one or more PEG-8 caprylic/capric glycerides is LABROSOL^{™} (available from Gattefossé, Lyon, France). Preferably, the source of one or more caprylocaproyl polyoxylglycerides and one or more PEG-8 caprylic/capric glycerides is LABROSOL^{™} ALF (Gattefossé).

In an embodiment, the oral formulation comprises LABROSOL^{™} (available from Gattefossé). In an embodiment, the oral formulation comprises LABROSOL^{™} ALF (Gattefossé).

The formulation can contain from about 0.1 to about 90 wt % of the pharmaceutically acceptable surfactant. In an embodiment, the formulation comprises from about 1 to about 90 wt % of the pharmaceutically acceptable surfactant. In an embodiment, the formulation comprises from about 50 to about 85 wt % of the pharmaceutically acceptable surfactant. In an embodiment, the formulation comprises from about 65 to about 85 wt % of the pharmaceutically acceptable surfactant. In an embodiment, the formulation comprises from about 75 to about 85 wt % of the pharmaceutically acceptable surfactant. In an embodiment, the formulation comprises about 80 wt % of the pharmaceutically acceptable surfactant.

The oral formulation can contain from about 0.1 to about 90 wt % of the pharmaceutically acceptable surfactant. In an embodiment, the oral formulation comprises from about 1 to about 90 wt % of the pharmaceutically acceptable surfactant. In an embodiment, the oral formulation comprises from about 50 to about 85 wt % of the pharmaceutically acceptable surfactant. In an embodiment, the oral formulation comprises from about 65 to about 85 wt % of the pharmaceutically acceptable surfactant. In an embodiment, the oral formulation comprises from about 75 to about 85 wt % of the pharmaceutically acceptable surfactant. In an embodiment, the oral formulation comprises about 80 wt % of the pharmaceutically acceptable surfactant.

The formulation can contain from about 0.1 to about 75 wt % of the pharmaceutically acceptable surfactant. In an embodiment, the formulation comprises from about 1 to about 70 wt % of the pharmaceutically acceptable surfactant. In an embodiment, the formulation comprises from about 25 to about 65 wt % of the pharmaceutically acceptable surfactant. In an embodiment, the formulation comprises from about 30 to about 60 wt % of the pharmaceutically acceptable surfactant. In an embodiment, the formulation comprises from about 40 to about 50 wt % of the pharmaceutically acceptable surfactant. In an embodiment, the formulation comprises about 45 wt % of the pharmaceutically acceptable surfactant.

The oral formulation can contain from about 0.1 to about 75 wt % of the pharmaceutically acceptable surfactant. In an embodiment, the oral formulation comprises from about 1 to about 70 wt % of the pharmaceutically acceptable surfactant. In an embodiment, the oral formulation comprises from about 25 to about 65 wt % of the pharmaceutically acceptable surfactant. In an embodiment, the oral formulation comprises from about 30 to about 60 wt % of the pharmaceutically acceptable surfactant. In an embodiment, the oral formulation comprises from about 40 to about 50 wt % of the pharmaceutically acceptable surfactant. In an embodiment, the oral formulation comprises about 45 wt % of the pharmaceutically acceptable surfactant.

The formulation comprising one or more caprylocaproyl polyoxylglycerides and one or more PEG-8 caprylic/capric glycerides can also contain caprylic acid. In an embodiment, the formulation comprises from about 0.1 to about 50 wt % of caprylic acid. In an embodiment, the formulation comprises from about 1 to about 50 wt % of caprylic acid. In an embodiment, the formulation comprises from about 3 to about 40 wt % of caprylic acid. In an embodiment, the formulation comprises from about 5 to about 30 wt % of caprylic acid. In an embodiment, the formulation comprises from about 5 to about 15 wt % of caprylic acid. In an embodiment, the formulation comprises about 10 wt % of caprylic acid.

**In** an embodiment, the formulation comprising one or more caprylocaproyl polyoxylglycerides and one or more PEG-8 caprylic/capric glycerides also comprises from about 15 to about 25 wt % of caprylic acid. In an embodiment, the formulation comprises about 20 wt % of caprylic acid.

The oral formulation comprising one or more caprylocaproyl polyoxylglycerides and one or more PEG-8 caprylic/capric glycerides can also contain caprylic acid. In an embodiment, the oral formulation comprises from about 0.1 to about 50 wt % of caprylic acid. In an embodiment, the oral formulation comprises from about 1 to about 50 wt % of caprylic acid. In an embodiment, the oral formulation comprises from about 3 to about 40 wt % of caprylic acid. In an embodiment, the oral formulation comprises from about 5 to about 30 wt % of caprylic acid. In an embodiment, the oral formulation comprises from about 5 to about 15 wt % of caprylic acid. In an embodiment, the oral formulation comprises about 10 wt % of caprylic acid.

In an embodiment, the oral formulation comprising one or more caprylocaproyl polyoxylglycerides and one or more PEG-8 caprylic/capric glycerides also comprises from about 15 to about 25 wt % of caprylic acid. In an embodiment, the oral formulation comprises about 20 wt % of caprylic acid.

The formulations comprising one or more caprylocaproyl polyoxylglycerides and one or more PEG-8 caprylic/capric glycerides can also contain water. The formulation can contain from about 0.1 to about 95 wt % water. In an embodiment, oral formulation comprises from about 0.1 to about 90 wt % water. In an embodiment, the formulation comprises from about 0.1 to about 85 wt % water. In an embodiment, the formulation comprises from about 0.1 to about 80 wt % water. In an embodiment, the formulation comprises from about 0.1 to about 75 wt % water. In an embodiment, the formulation comprises from about 1 to about 70 wt % water. In an embodiment, the formulation comprises from about 25 to about 65 wt % water. In an embodiment, the formulation comprises from about 30 to about 60 wt % water. In an embodiment, the formulation comprises from about 40 to about 50 wt % water. In an embodiment, the formulation comprises about 45 wt % water.

The oral formulations comprising one or more caprylocaproyl polyoxylglycerides and one or more PEG-8 caprylic/capric glycerides can also contain water. The oral formulation can contain from about 0.1 to about 95 wt % water. In an embodiment, the oral formulation comprises from about 0.1 to about 90 wt % water. In an embodiment, the oral formulation comprises from about 0.1 to about 85 wt % water. In an embodiment, the oral formulation comprises from about 0.1 to about 80 wt % water. In an embodiment, the oral formulation comprises from about 0.1 to about 75 wt % water. In an embodiment, the oral formulation comprises from about 1 to about 70 wt % water. In an embodiment, the oral formulation comprises from about 25 to about 65 wt % water. In an embodiment, the oral formulation comprises from about 30 to about 60 wt % water. In an embodiment, the oral formulation comprises from about 40 to about 50 wt % water. In an embodiment, the oral formulation comprises about 45 wt % water.

In addition to the pharmaceutically acceptable surfactant, the formulations suitable for oral administration can include (a) liquid solutions, such as a therapeutically effective amount of the compound dissolved in diluents (e.g., water, saline, or juice), (b) capsules, sachets, tablets, lozenges, and troches, each containing a predetermined amount of the active ingredient, as solids or granules, (c) powders, (d) suspensions in an appropriate liquid, and (e) suitable emulsions. Liquid formulations may include diluents, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, additional surfactants, lubricants, and inert fillers, such as lactose, sucrose, calcium phosphate, and corn starch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Preferably, the formulation comprising one or more caprylocaproyl polyoxylglycerides and one or more PEG-8 caprylic/capric glycerides is an oral formulation and is a suspension.

Preferably, the formulation comprising one or more caprylocaproyl polyoxylglycerides and one or more PEG-8 caprylic/capric glycerides is an oral formulation and is a pill. A pill can be a tablet or capsule (hard or soft).

An embodiment of the invention provides a formulation comprising a pharmaceutically acceptable surfactant comprising one or more polyoxyethylene esters of 12-hydroxystearic acid. In an embodiment, the formulation is administered by any one of the following methods: oral, aerosol, nasal, pulmonary, parenteral (e.g., IV, subcutaneously, intradermally, or intramuscularly), subcutaneous, intravenous, intramuscular, intraperitoneal, intrathecal, intratumoral, topical, rectal, and vaginal. Preferably, the formulation is suitable for intravenous administration.

A suitable source of one or more polyoxyethylene esters of 12-hydroxystearic acid is Solutol HS 15.

In an embodiment, the formulation comprises from about 1 to about 60 wt % of one or more polyoxyethylene esters of 12-hydroxystearic acid. In an embodiment, the formulation comprises from about 5 to about 55 wt % of one or more polyoxyethylene esters of 12-hydroxystearic acid. In an embodiment, the formulation comprises from about 10 to about 50 wt % of one or more polyoxyethylene esters of 12-hydroxystearic acid. In an embodiment, the formulation comprises from about 15 to about 45 wt % of one or more polyoxyethylene esters of 12-hydroxystearic acid. In an embodiment, the formulation comprises from about 20 to about 40 wt % of one or more polyoxyethylene esters of 12-hydroxystearic acid. In an embodiment, the formulation comprises from about 25 to about 35 wt % of one or more polyoxyethylene esters of 12-hydroxystearic acid. In an embodiment, the formulation comprises about 30 wt % of one or more polyoxyethylene esters of 12-hydroxystearic acid.

In an embodiment, the IV formulation comprises from about 1 to about 60 wt % of one or more polyoxyethylene esters of 12-hydroxystearic acid. In an embodiment, the IV formulation comprises from about 5 to about 55 wt % of one or more polyoxyethylene esters of 12-hydroxystearic acid. In an embodiment, the IV formulation comprises from about 10 to about 50 wt % of one or more polyoxyethylene esters of 12-hydroxystearic acid. In an embodiment, the IV formulation comprises from about 15 to about 45 wt % of one or more polyoxyethylene esters of 12-hydroxystearic acid. In an embodiment, the IV formulation comprises from about 20 to about 40 wt % of one or more polyoxyethylene esters of 12-hydroxystearic acid. In an embodiment, the IV formulation comprises from about 25 to about 35 wt % of one or more polyoxyethylene esters of 12-hydroxystearic acid. In an embodiment, the IV formulation comprises about 30 wt % of one or more polyoxyethylene esters of 12-hydroxystearic acid.

In an embodiment, the formulation comprises from about 1 to about 60 wt % Solutol HS 15. In an embodiment, the formulation comprises from about 5 to about 55 wt % Solutol HS 15. In an embodiment, the formulation comprises from about 10 to about 50 wt % Solutol HS 15. In an embodiment, the formulation comprises from about 15 to about 45 wt % Solutol HS 15. In an embodiment, the formulation comprises from about 20 to about 40 wt % Solutol HS 15. In an embodiment, the formulation comprises from about 25 to about 35 wt % Solutol HS 15. In an embodiment, the formulation comprises about 30 wt % Solutol HS 15.

In an embodiment, the IV formulation comprises from about 1 to about 60 wt % Solutol HS 15. In an embodiment, the IV formulation comprises from about 5 to about 55 wt % Solutol HS 15. In an embodiment, the IV formulation comprises from about 10 to about 50 wt % Solutol HS 15. In an embodiment, the IV formulation comprises from about 15 to about 45 wt % Solutol HS 15. In an embodiment, the IV formulation comprises from about 20 to about 40 wt % Solutol HS 15. In an embodiment, the IV formulation comprises from about 25 to about 35 wt % Solutol HS 15. In an embodiment, the IV formulation comprises about 30 wt % Solutol HS 15.

Preferably, the IV formulation comprises water.

Preferably, the IV formulation comprises saline.

### Methods of Treatment

Any references to methods of treatment by therapy or surgery or in vivo diagnosis methods of this description is to be interpreted as a reference to compounds, pharmaceutical compositions and medicaments of the present invention for use in those methods. A embodiment of the invention provides formulations as described before for use in treating pancreatic adenocarcinoma in the mammal.

Also disclosed is a method which comprises administering the compounds of formula (I) with another treatment. The additional treatment can be a radiation treatment. The radiation treatment can be any suitable radiation treatment used in the treatment of pancreatic adenocarcinoma.

The additional treatment can be a chemotherapeutic agent. The chemotherapeutic agent can be any suitable chemotherapeutic agent, for example, the chemotherapeutic agent can be selected from the group consisting of asparaginase, busulfan, carboplatin, cisplatin, daunorubicin, doxorubicin, fluorouracil, gemcitabine, hydroxyurea, methotrexate, paclitaxel, rituximab, vinblastine, and vincristine. Preferably, the chemotherapeutic agent is gemcitabine.

The chemotherapeutic agent can be administered sequentially with a compound of formula (I). The chemotherapeutic agent can be administered before a compound of formula (I). The chemotherapeutic agent can be administered after a compound of formula (I).

Gemcitabine can be administered sequentially with a compound of formula (I). Gemcitabine can be administered before a compound of formula (I). Gemcitabine can be administered after a compound of formula (I).

The chemotherapeutic agent can be administered simultaneously with a compound of formula (I).

Gemcitabine can be administered simultaneously with a compound of formula (I).

When administered, the compounds of formula (I) reduce the likelihood that pancreatic cancer will metastasize. If the tumor has already metastasized, then administration of the compounds of formula (I) may reduce the number or volume of metastatic tumors. In an embodiment, the mammal has stage I pancreatic adenocarcinoma. In an embodiment, the mammal has stage II pancreatic adenocarcinoma. In an embodiment, the mammal has stage III pancreatic adenocarcinoma. In an embodiment, the mammal has stage IV pancreatic adenocarcinoma.

In an embodiment, the mammal has metastatic pancreatic adenocarcinoma.

In a further embodiment, administration of the compound of formula (I) reduces or delays further metastasizing of established metastases. Further, administration of the compounds of formula (I) may reduce the amount or size of metastases (e.g., by about 90%, about 80%, about 70%, about 60%, about 50%, about 40%, about 30%, about 20%, about 10%, or about 5%).

The compounds of formula (I) may be administered before one or more pancreatic adenocarcinoma tumor(s) has been removed from the mammal. The compounds of formula (I) may be administered after one or more pancreatic adenocarcinoma tumor(s) has been removed from the mammal. The tumor(s) may be removed, for example, by surgery.

In an embodiment, the methods of treatment result in the disrupting of a perinucleolar compartment in a cell in the mammal.

In an embodiment, the methods of treatment result in reducing the prevalence of perinucleolar compartment in a cell in the mammal.

In an embodiment, the methods of treatment result in reducing adenosine triphosphate (ATP) levels produced by metastatic cancer cells in the mammal.

In an embodiment, the methods of treatment result in reducing the colony formation of cancer cells in the mammal.

In an embodiment, the methods of treatment result in reducing the migration of cancer cells in the mammal.

The term "treat," as well as words stemming therefrom, as used herein, does not necessarily imply 100% or complete treatment. Rather, there are varying degrees of treatment of which one of ordinary skill in the art recognizes as having a potential benefit or therapeutic effect. In this respect, the inventive methods can provide any amount of any level of treatment of the cancer in a mammal. Furthermore, the treatment provided by the inventive method can include treatment of one or more conditions or symptoms of the cancer being treated or prevented. For example, treatment can include promoting the regression of a tumor.

### Dosing

The therapeutically effective amount of the compound administered can vary depending upon the desired effects and the factors noted above. In an embodiment, the dosages will be between 0.1 mg/kg and 80 mg/kg of the subject's body weight. In an embodiment, the dosages will be between 0.1 mg/kg and 70 mg/kg of the subject's body weight. In an embodiment, the dosages will be between 0.1 mg/kg and 60 mg/kg of the subject's body weight. In an embodiment, the dosages will be between 0.1 mg/kg and 50 mg/kg of the subject's body weight. In an embodiment, the dosages will be between 0.1 mg/kg and 40 mg/kg of the subject's body weight. In an embodiment, the dosages will be between 0.1 mg/kg and 30 mg/kg of the subject's body weight. In an embodiment, the dosages will be between 0.1 mg/kg and 20 mg/kg of the subject's body weight. In an embodiment, the dosages will be between 0.1 mg/kg and 10 mg/kg of the subject's body weight. In an embodiment, the dosages will be between 0.1 mg/kg and 9 mg/kg of the subject's body weight. In an embodiment, the dosages will be between 0.1 mg/kg and 8 mg/kg of the subject's body weight. In an embodiment, the dosages will be between 0.1 mg/kg and 7 mg/kg of the subject's body weight. In an embodiment, the dosages will be between 0.1 mg/kg and 6 mg/kg of the subject's body weight. In an embodiment, the dosages will be between 0.1 mg/kg and 5 mg/kg of the subject's body weight. In an embodiment, the dosages will be between 0.1 mg/kg and 4 mg/kg of the subject's body weight. In an embodiment, the dosages will be between 0.1 mg/kg and 3 mg/kg of the subject's body weight. In an embodiment, the dosages will be between 0.1 mg/kg and 2 mg/kg of the subject's body weight. In an embodiment, the dosages will be between 0.1 mg/kg and 1 mg/kg of the subject's body weight.

In an embodiment, the dosages will be between 0.5 mg/kg and 1.5 mg/kg of the subject's body weight. In an embodiment, the dosages will be between 0.8 mg/kg and 1.2 mg/kg of the subject's body weight. In an embodiment, the dosages will be about 1 mg/kg of the subject's body weight.

In an embodiment, the dosages will be between 1.5 mg/kg and 2.5 mg/kg of the subject's body weight. In an embodiment, the dosages will be between 1.8 mg/kg and 2.2 mg/kg of the subject's body weight. In an embodiment, the dosages will be about 2 mg/kg of the subject's body weight.

In an embodiment, the dosages will be between 4.5 mg/kg and 5.5 mg/kg of the subject's body weight. In an embodiment, the dosages will be between 4.8 mg/kg and 5.2 mg/kg of the subject's body weight. In an embodiment, the dosages will be about 5 mg/kg of the subject's body weight.

The doses disclosed herein can be administered daily. The dose can be administered at one time per day or more than one time per day. The dose can be administered simultaneously or sequentially with other treatments.

### Mammals

The methods herein comprise administering an effective amount of the compound of formula (I) to an animal afflicted with pancreatic adenocarcinoma. Preferably, the animal is a mammal. More preferably, the mammal is a human.

The term "mammal" includes, but is not limited to, the order Rodentia, such as mice, and the order Logomorpha, such as rabbits. It is preferred that the mammals are from the order Carnivora, including Felines (cats) and Canines (dogs). It is more preferred that the mammals are from the order Artiodactyla, including Bovines (cows) and Swines (pigs) or of the order Perssodactyla, including Equines (horses). It is most preferred that the mammals are of the order Primates, Ceboids, or Simioids (monkeys) or of the order Anthropoids (humans and apes). An especially preferred mammal is the human. Furthermore, the subject can be the unborn offspring of any of the forgoing hosts, especially mammals (such as, humans), in which case any screening of the subject or cells of the subject, or administration of compounds to the subject or cells of the subject, can be performed in utero.

### Biomarkers

The disclosure provides methods for detecting the change in expression levels of one or both of FoxA1 and FoxO6 in a pancreatic adenocarcinoma tumor sample from a mammal, wherein the mammal has been administered a compound of formula (I). The method involves the steps of providing a first pancreatic adenocarcinoma tumor sample from the mammal, assaying the tumor sample to determine the expression levels of one or both of FoxAl and FoxO6, providing a second pancreatic adenocarcinoma tumor sample from the mammal, assaying the second tumor sample to determine the expression levels of one or both of FoxA1 and FoxO6, and comparing one or both of (i) the first determined expression level of FoxA1 to the second determined level of FoxA1 and (ii) comparing the first determined expression level of FoxO6 and the second determined level of FoxO6, to detect a change in expression levels of one or both of FoxA1 and FoxO6, wherein the first tumor sample is removed from the mammal before the second tumor sample is removed from the mammal.

FoxA1 is also referred to as hepatocyte nuclear factor 3-alpha (HNF-3A). FoxA1 is encoded by the gene FOXA1 in humans. The sequence of FOXA1 is publicly available (see, for example, NCBI's database, Gene ID: 3169).

FoxO6 is encoded by the gene FOXO6 in humans. The sequence of FOXO6 is publicly available (see, for example, NCBI's database, Gene ID: 100132074).

**In** an embodiment, the method comprises obtaining a first sample from the subject. In an embodiment, the first sample is a pancreatic adenocarcinoma tissue sample. Obtaining a first sample from the subject may be carried out in any suitable manner known in the art, and the sample may be from any suitable source, for example, from tumor resection material or a tumor biopsy (i.e., gross biopsy or fine needle biopsy).

**In** an embodiment, the method comprises obtaining a second sample from the subject. In an embodiment, the second sample is a pancreatic adenocarcinoma tissue sample. Obtaining a second sample from the subject may be carried out in any suitable manner known in the art, and the sample may be from any suitable source, for example, from tumor resection material or a tumor biopsy (i.e., gross biopsy or fine needle biopsy).

The first and second samples may be obtained by different methods. The first and second samples may be obtained from different parts of the tumor, or different parts of the organ.

In an embodiment, the method comprises assaying the first sample and the second sample to detect the levels of FoxA1 in the samples. For example, the FoxA1 protein can be purified from the samples (either partially or substantially) and assayed via immunohistological techniques (e.g., Western blotting, ELISA, immunoprecipitation, etc.) using one or more antibodies recognizing FoxA1 protein. In this regard, the assaying may comprise contacting the samples with an antibody that specifically binds to FoxA1 protein and thereby forming a complex, and detecting the complex. In an embodiment, the first sample and second sample are purified and assayed via the same or similar techniques.

In an embodiment, the FoxA1 protein comprises an amino acid sequence of SEQ ID NO: 19. In an embodiment, the FoxA1 protein can be at least about 30%, about 50%, about 75%, about 80%, about 90%, about 91%, about 92%, about 93%, about 94%, about 95%, about 96%, about 97%, about 98%, about 99%, or about 100%identical to SEQ ID NO: 19.

In an embodiment, the method comprises assaying the first sample and the second sample to detect the levels of FOXO6 in the samples. For example, the FoxO6 protein can be purified from the samples (either partially or substantially) and assayed via immunohistological techniques (e.g., Western blotting, ELISA, immunoprecipitation, etc.) using one or more antibodies recognizing FoxO6 protein. In this regard, the assaying may comprise contacting the sample with an antibody that specifically binds to FoxO6 protein and thereby forming a complex, and detecting the complex. In an embodiment, the first sample and second sample are purified and assayed via the same or similar techniques.

In an embodiment, the FoxO6 protein comprises an amino acid sequence of SEQ ID NO: 20. In an embodiment, the FoxO6 protein can be at least about 30%, about 50%, about 75%, about 80%, about 90%, about 91%, about 92%, about 93%, about 94%, about 95%, about 96%, about 97%, about 98%, about 99%, or about 100% identical to SEQ ID NO: 20.

By assaying the first sample and second sample, the change in the level of the biomarker expression can be determined. In an embodiment, the first sample is taken from the mammal before a compound of formula (I) is administered to the mammal. In an embodiment, the level of one or both biomarkers in the second sample is compared to the mammal's pre-treatment level of each biomarker (which is known by assaying the first sample).

In an embodiment, the first sample is taken from the mammal within 1 week of the first administration of a compound of formula (I). In an embodiment, the first sample is taken from the mammal within 2 weeks of the first administration of a compound of formula (I).

In an embodiment, the first sample is taken from the mammal at least 1 week before the second sample is taken from the mammal. In an embodiment, the first sample is taken from the mammal at least 2 weeks before the second sample is taken from the mammal.

In an embodiment, the first sample is taken from the mammal at least 3 weeks before the second sample is taken from the mammal. In an embodiment, the first sample is taken from the mammal at least 4 weeks before the second sample is taken from the mammal. In an embodiment, the first sample is taken from the mammal at least 5 weeks before the second sample is taken from the mammal. In an embodiment, the first sample is taken from the mammal at least 6 weeks before the second sample is taken from the mammal. In an embodiment, the first sample is taken from the mammal at least 7 weeks before the second sample is taken from the mammal. In an embodiment, the first sample is taken from the mammal at least 8 weeks before the second sample is taken from the mammal.

If the mammal's level of FoxA1 expression in the second sample is below the level of FoxA2 expression in the first sample, then the mammal is predicted to have a favorable response to the compound of formula (I) (fewer or smaller metastatic tumors) compared to not being administered the compound of formula (I). If the mammal's level of FoxO6 expression in the second sample is above the level of FoxO6 expression in the first sample, then the mammal is predicted to have a favorable response to the compound of formula (I) (fewer or smaller metastatic tumors) compared to not being administered the compound of formula.

In an embodiment, the methods of treating pancreatic adenocarcinoma comprise receiving a determination of the mammal as having a lower level of FoxA1 and/or a higher level of FoxO6 expression in the second sample compared to the first sample, wherein the lower level of FoxA1 or the higher level of FoxO6 expression has been determined by a method according to an embodiment of the invention, predicting the clinical response to the administration of the compound of formula (I), and treating the mammal for pancreatic adenocarcinoma by administering a compound of formula (I) to the mammal if the mammal has a lower level of FoxA1 and/or a higher level of FoxO6 expression in the second sample as compared to the first sample.

As used herein, the phrase "predicting the clinical response" refers to determining whether the number or size or volume of metastatic tumors would likely decrease in a subject following administration of a compound of formula (I).

When claimed herein, the term "biomarker" refers to FoxA1 or FoxO6.

### Compounds of Formula (I)

The phrase "pharmaceutically acceptable salt" is intended to include non-toxic salts synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media such as ether, ethyl acetate, ethanol, isopropanol, or acetonitrile are preferred. Lists of suitable salts are found in Remington's Pharmaceutical Sciences, 22nd ed., Pharmaceutical Press, (2012).

Suitable bases include inorganic bases such as alkali and alkaline earth metal bases, such as those containing metallic cations such as sodium, potassium, magnesium, calcium and the like. Non-limiting examples of suitable bases include sodium hydroxide, potassium hydroxide, sodium carbonate, and potassium carbonate. Suitable acids include inorganic acids such as hydrochloric acid, hydrobromic acid, hydroiodic acid, sulfuric acid, phosphoric acid, and the like, and organic acids such as *p*-toluenesulfonic, methanesulfonic acid, benzenesulfonic acid, oxalic acid, p-bromophenylsulfonic acid, carbonic acid, succinic acid, citric acid, benzoic acid, acetic acid, maleic acid, tartaric acid, fatty acids, long chain fatty acids, and the like. Preferred pharmaceutically acceptable salts of compounds having an acidic moiety include sodium and potassium salts. Preferred pharmaceutically acceptable salts of compounds having a basic moiety (such as a dimethylaminoalkyl group) include hydrochloride and hydrobromide salts. The compounds containing an acidic or basic moiety are useful in the form of the free base or acid or in the form of a pharmaceutically acceptable salt thereof.

It should be recognized that the particular counterion forming a part of any salt of this invention is usually not of a critical nature, so long as the salt as a whole is pharmacologically acceptable and as long as the counterion does not contribute undesired qualities to the salt as a whole.

It is further understood that the above compounds and salts may form solvates, or exist in a substantially uncomplexed form, such as the anhydrous form. As used herein, the term "solvate" refers to a molecular complex wherein the solvent molecule, such as the crystallizing solvent, is incorporated into the crystal lattice. When the solvent incorporated in the solvate is water, the molecular complex is called a hydrate. Pharmaceutically acceptable solvates include hydrates, alcoholates such as methanolates and ethanolates, acetonitrilates and the like. These compounds can also exist in polymorphic forms.

In any of the above embodiments, the compound or salt of formula (I) can have at least one asymmetric carbon atom. When the compound or salt has at least one asymmetric carbon atom, the compound or salt can exist in the racemic form, in the form of its pure optical isomers, or in the form of a mixture wherein one isomer is enriched relative to the other. In particular, in accordance with the present invention, when the compounds have a single asymmetric carbon atom, the compounds may exist as racemates, that is as mixtures of equal amounts of optical isomers, that is equal amounts of two enantiomers, or in the form of a single enantiomer. As used herein, "single enantiomer" is intended to include a compound that comprises more than 50% of a single enantiomer (that is enantiomeric excess up to 100% pure enantiomer).

When the compound or salt has more than one chiral center, the compound or salt can therefore exist as a mixture of diastereomers or in the form of a single diastereomer. As used herein, "single diastereomer" is intended to mean a compound that comprises more than 50% of a single diastereomer (that is diastereomeric excess to 100% pure diastereomer).

The following examples further illustrate the invention but, of course, should not be construed as in any way limiting its scope.

### EXAMPLES

### EXAMPLE 1

This example explains how the compounds of formula (I) were identified using PNC as a phenotypic marker and surrogate of metastatic behavior.

A metastatic prostate cancer cell line, PC3M, was developed in order to screen small molecules that effectively disassemble PNCs (see Pettaway, et al., "Selection of highly metastatic variants of different human prostatic carcinomas using orthotopic implantation in nude mice," Clin. Cancer Res., (2): 1627-36 (1996)). PC3M has a PNC prevalence of 75-85% to stably express GFP-PTB (polypyrimidine track binding protein) (Fig. 1, top panels), an essential component of PNCs (see Huang, et al., "The perinucleolar compartment and transcription," Journal of Cell Biology, (143): 35-47 (1998)). Compounds able to reduce PNC prevalence by 50% were filtered in secondary assays to eliminate those that induced apoptosis, DNA damage, generic cytotoxicity, or cell-cycle blockage (see Norton, et al., "Automated high-content screening for compounds that disassemble the perinucleolar compartment," J Biomol Screen, (14): 1045-53 (2009) and Frankowski, et al., "Discovery and Development of Small Molecules That Reduce PNC Prevalence, in Probe Reports from the NIH Molecular Libraries Program, Bethesda (MD) (2010)). The remaining hits were evaluated for invasion inhibition through MATRIGEL gelatinous protein mixture and anchorage-independent growth inhibition using soft agar assays.

24-well Transwell Permeable MATRIGEL gelatinous protein mixture Invasion assay: The effect of metarrestin on invasion activity of PANC1 and PC3M cells was measured using 24-well Transwell Permeable MATRIGEL gelatinous protein mixture Invasion Chambers with 8 µm pores (Corning, Cat #354480). Membranes were rehydrated with 500 µL of serum-free DMEM (Thermo Fisher, Cat #11965084) both inside the chamber and inside the well and incubated for two hours at 37 °C, 5% CO₂. Cells were detached, washed, resuspended in serum-free DMEM, counted, and diluted to a final concentration of 1 × 10⁵ cells/mL in six separate 15 mL conical tubes. Metarrestin or vehicle was added to each tube in decreasing concentrations, as indicated in Fig. 3, and 500 µL of resuspended cells in metarrestin or vehicle-containing medium were placed in the upper chambers in triplicates. 750 µL of DMEM plus 10% FBS acting as a chemoattractant were placed in the basal wells. Plates were incubated at 37 °C, 5% CO₂ for 24 hours. To count the number of invading cells, medium from the top insert was removed and a cotton swab was used to wipe out non-invading cells from the top side of the membrane on the insert. Invading cells on the bottom of the membrane on the insert were fixed and stained using the three-step DIFF QUIK Rapid Stain Set (Siemens) followed by three water washes and air drying. Cell invasion for each compound treatment was performed in triplicate. Representative photographs were taken of each insert.

A compound, named MLS000556915, was selected for further study based on its potency as a PNC inhibitor, soft agar growth inhibitor, ability to block invasion, and lack of cytotoxicity.

Based on the screening results, additional medicinal chemistry studies were then used to design a compound of formula (I), metarrestin (structure below).

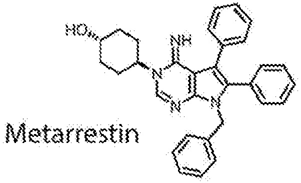

Throughout the examples and figures, data are presented as means +/- SD. Student's t tests were performed for all the experiments, except where indicated. Mean nucleolar areas were analyzed using Mann Whitney U test, 2-tailed.

### EXAMPLE 2

This example shows that a compound of formula (I), metarrestin, disrupts the PNC in cancer cell types.

In summary, several cell types (see list below in Table 1) were treated with 1 µM (IC100 for PC3M cells) of metarrestin for 24 hours. Specifically, PC-3M cells were maintained in RPMI 1640 medium. Other cell lines including HeLa, Panc1, PACADD159, PACADD183, PA-TU-8988T, KP-3, PK-8, NOR-P1, L3.6sl, Colo357, Suit-2, HPAC were maintained in DMEM with 10% FBS (GEMINI^{™} Bio Products) and 100 units/mL of penicillin and streptomycin. Cells were generally plated approximately 24 h before treatment with metarrestin for 5 h or 24 h at 1 µM.

**Table 1. Cells lines tested**

| **Cell Line** | **Origin** |
|---|---|
| T47D | breast cancer |
| CG | neuroblastoma |
| TKF-1 | cholangiocellular carcinoma |
| Panc1 | pancreatic ductal carcinoma |
| 83 Mer | neuroblastoma cancer stem cells |
| PACADD159 | pancreatic carcinoma |
| KP-3 | adenosquamous carcinoma of the human pancreatic duct |
| SKOV3 | ovarian adenocarcinoma |
| PACADD183 | pancreas carcinoma from ascites |
| PA-TU-8988T | liver metastasis of a primary pancreatic adenocarcinoma |
| HEK293T | 293 cells expressing a mutant version of the SV40 large T antigen |
| NOR-P1 | metastatic subcutaneous tumor of a patient with pancreatic cancer |
| colo205 | colorectal carcinoma |
| L3.6sl | liver metastasis from injecting COLO-357 cells into mouse spleen |
| PC-3M | human prostate carcinomas |
| HeLa | cervix adenocarcinoma |
| Colo357 | liver metastasis of pancreatic adenocarcinoma |
| Suit-2 | liver metastasis of pancreatic carcinoma |
| HPAC | pancreatic adenocarcinoma cell line from a nude mouse xenograft of a primary tumor |

Surprisingly, it was found that metarrestin reduced PNC prevalence in different human cancer cell lines (Fig. 2). For example, metarrestin disrupted PNCs in PC3M-GFP-PTB cells with an IC₅₀ of 0.39 µM (Fig. 1, bottom graph, squares) without acute cytotoxicity (Fig. 1, bottom graph, circles). Further, analysis of the MATRIGEL gelatinous protein mixture invasion assays showed that metarrestin effectively blocked the invasion of PC3M and PANC1 cells (Fig. 3) within 24 hours at a concentration (0.6 µM) which did not affect PC3M or PANC1 cell growth after 24 hours of treatment (Fig. 4 for PC3M cells, and Fig. 46 for PANC1 cells). When PC3M cells and normal human fibroblasts (GM02153) were treated at 1 µM concentration for an extended time using the INCUCYTE^{™} system, metarrestin preferentially inhibited cell growth in the PC3M cancer cells (Fig. 4).

### EXAMPLE 3

This example demonstrates that a compound of formula (I), metarrestin, suppresses metastasis and extends survival in mouse models of human pancreatic cancer xenografts.

Because pancreatic cancer patients suffer particular high mortality from metastasis, the *in vivo* efficacy of metarrestin against metastasis in an orthotopic pancreatic cancer metastasis model was evaluated. The 3D PANC1 cell spheres model, deployed in NOD/interleukin 2 receptor common gamma chain (*Il2rγ)^{null}* (NSG) PANC-1 mice (see Suemizu et al., "Identification of a key molecular regulator of liver metastasis in human pancreatic carcinoma using a novel quantitative model of metastasis in NOD/SCID/gammacnull (NOG) mice," Int. J. Oncol., (31): 741-51 (2007), ecapitulates human cancer progression and metastatic phenotype of the disease without the limitations of early death due to complications of local invasion, such as gastric outlet obstruction or impingement of the common bile duct. Sixty thousand 3D luciferase-expressing PANC1-luc cell spheres were injected orthotopically into the pancreas of NSG mice. Histopathological examination revealed that measurable metastasis, in the form of occasional peri-portal infiltrates and micrometastatic deposits, had developed in livers approximately 4 weeks after implantation in the form of parenchymal infiltration. By 8 weeks, macrometastasis on the liver surface were visible. Mice had a lifespan of 10-14 weeks (see Fig. 63).

The PNC prevalence was measured in a panel of pancreatic cancer cell lines derived from primary tumors and metastatic lesions from various organs in NSG PANC1 mice to determine whether the preclinical model used retained the features of PNCs observed previously in clinical specimens. The results showed that PNCs were more numerous in human cancer lineages from a metastatic origin and in metastatic lesions compared to those from primary tumors (Fig. 5, cell line explanation, see Table 2 below). When cryopreserved tissue sections were examined, PNCs were detected by immunolabeling with a monoclonal anti-human specific PTB antibody, SH54, which labels PNCs and allows for specific identification of human xenograft tissues over mouse tissues. PNC prevalence was higher in metastatic lesions than in primary tumors harvested 8 weeks after implantation (Fig. 6).

**Table 2: Pancreas cancer cell lines evaluated for PNC prevalence**

| Cell Lines | (1) Primary | PNC prevalence (%) | Detailed origin |
|---|---|---|---|
| | (2) Met | | |
| Panc 8.13 | 1 | 3 | Primary tissue culture line |
| SB06 | 1 | 7 | LN met |
| SB.07 | 1 | 13 | primary - distal pancreatectomy |
| Panc3.27 | 1 | 17 | Primary tissue culture line |
| Panc10.05 | 1 | 30 | Primary tissue culture line |
| PSN1 | 1 | 3 | patient-derived xenograft - primary |
| KLM1 | 1 | 22 | primary |
| KP-3 | 1 | 50 | patient-derived xenograft - primary |
| SU86 | 1 | 8 | pancreas (not SU86.86, not obtained from ATCC) primary explant |
| L3.3 | 2 | 50 | COLO357 propagated through pancreas in nude mice |
| YAPC | 2 | 10 | explant from ascites |
| L3.6pl | 2 | 55 | COLO357 propagated through liver in nude mice |
| L3.6sl | 2 | 80 | COLO357 propagated through spleen in nude mice |
| Colo357 | 2 | 93 | liver met |
| PK-8 | 2 | 55 | liver met |
| PATU-8988T | 2 | 58 | liver met |
| NOR-P1 | 2 | 70 | subcutaneous met |
| SUIT-2 | 2 | 97 | liver met |

### EXAMPLE 4

This example demonstrates that a compound of formula (I), metarrestin, has a favorable PK profile and that administration of metarrestin successfully suppresses metastasis-related death in metarrestin-treated mice.

This study (and those that follow) were randomized, unblinded, and conducted in accordance with the *NIH Guide for the Care and Use of Laboratory Animals.* The animals were sacrificed at the predetermined time point or upon signs of animal distress (immobility or weight loss greater than 15 %). Animal data were excluded as outliers in cases of unspecified death or sacrifice prior to the predetermined endpoint, which affected less than 10 % of initially enrolled mice.

BALB/c mice, 17-19 g, female, n = 66, were purchased and had free access to food and water. They were dosed with metarrestin at 5 or 25 mg/kg (10 ml/kg) in 5% NMP [NMP (Sigma-Aldrich cat#270458), 20% PEG400 (Sigma-Aldrich cat#202398), and 75% 10% HPBCD (Sigma-Aldrich cat# H107)] via lower left abdominal quadrant injection (n = 33 each dose). The animals were anesthetized with isoflurane and restrained manually at the designated time points. Approximately 120 µL of blood were taken from the animals into K₂EDTA tubes via retro-orbital puncture. Blood samples were placed on ice and centrifuged (2,000 g, 5 min under 4 °C) to obtain plasma samples within 15 minutes. An aliquot of 20 µL plasma sample was protein-precipitated with 200 µL acetonitrile that contained 50 ng/mL IS (dexamethasone). The mixture was vortexed for 2 min, centrifuged at 14,000 rpm for 5 min, and an aliquot of 30 µL supernatant was diluted with 70 µL MeOH:H₂O (1:1,v/v), then vortexed for 2 min. A 2 µL aliquot of supernatant was injected into UPLC-MS/MS. Analysis of samples was done with an LC-MS/MS-02 (API4000), Waters-BEH-C18 (2.1 × 50 mm, 1.7 µm) column, flow rate 0.6 mL/min, with a mobile phase consisting of solvent A (H₂O- 0.025%FA-1 mM NH₄OAc) and solvent B (MeOH- 0.025%FA-1 mM NH₄OAc). The gradient was 10, 90, 90, 10% B at 0.30, 0.60, 1.20, 1.21, 1.80 (stop) min. Retention times for metarrestin and dexamethasone were 1.09 and 1.08 min, respectively. Calibration curves were established with known serially diluted concentrations of metarrestin before analysis. SCID Beige mice were purchased and dosed with metarrestin at 5 and 25 mg/kg, once daily at 24 hour intervals for 4 weeks as above. Twenty-four hours after the final dose, the concentrations of metarrestin in plasma were analyzed.

PK studies using single and multiple daily dosing via IP administration of metarrestin in mice (Fig. 46) at 5 and 25 mg/kg indicated good exposure, distribution, and tolerability in vivo, with a half-life of 4.6 to 5.5 hours and a predicted moderate risk of accumulation (Fig. 46 and Tables 3 and 4). Metarrestin showed high bioavailability, with concentrations in plasma above its PNC disassembling IC₅₀ of 0.39 M in P3CM cells for an extended period of time (Fig. 1 and Fig. 46), and concentrations more than 10-fold above the IC₉₀ of 0.75 µM (Fig. 1) in primary tumors and even higher in metastatic deposits of tumor-bearing NSG PANC1 animals 1 hour after discontinuation of 10 mg/kg metarrestin given for 7 days via PO gavage (see Table 5 below).

**Table 3. Metarrestin Plasma PK - Single Dose**

| **Single dose pharmacokinetics in Plasma** | | | | |
|---|---|---|---|---|
| | 5 mg/kg IP | | 25 mg/kg IP | |
| Sampling time (h) | ng/mL | mM | ng/mL | mM |
| 0.083 | 308 | 0.649 | 2180 | 4.593 |
| 0.25 | 430 | 0.906 | 2660 | 5.605 |
| 0.5 | 429 | 0.904 | 2930 | 6.174 |
| 1 | 391 | 0.824 | 2680 | 5.647 |
| 2 | 217 | 0.457 | 2000 | 4.214 |
| 3 | 235 | 0.495 | 1630 | 3.434 |
| 4 | 221 | 0.466 | 1563 | 3.293 |
| 8 | 119 | 0.251 | 1065 | 2.244 |
| 12 | 69.3 | 0.146 | 818 | 1.724 |
| 24 | 11.1 | 0.023 | 279 | 0.588 |
| 48 | BQL | BQL | 5.09 | 0.011 |

**Table 4. Metarrestin Plasma PK - Repeat Dose**

| **Repeat dose pharmacokinetics in Plasma** | | | | |
|---|---|---|---|---|
| | 5 mg/kg IP | | 25 mg/kg IP | |
| Sampling time (h) | ng/mL | mM | ng/mL | mM |
| 24 | 48.4 | 0.102 | 692 | 1.458 |

**Table 5. Metarrestin Tumor PK**

| **Animal** | **Pancreas primary tumor (µmol/kg)** | **Liver metastasis (µmol/kg)** |
|---|---|---|
| 1 | 49.5 | 87.2 |
| 2 | 55.2 | 79 |
| 3 | 20.6 | 117.2 |
| 4 | 20.4 | 43.2 |
| 5 | 34.1 | 64.6 |
| 6 | 63 | 149.6 |
| 7 | 23.6 | 59.8 |

Four weeks after inoculation, mice were treated once daily with metarrestin (5 mg/kg or 25 mg/kg) or vehicle via IP injections, continuing for six weeks. At the end of the tenth week after initial inoculation, the cohort exposed to daily administration of 25 mg/kg metarrestin displayed a decrease in metastatic burden in both liver (p <0.01) and lung (p <0.05) compared to vehicle-treated control as measured by Xenogen photon organ/tumor ratios (Fig. 7) and by standard histopathological examination (Fig. 8 and 9, p <0.001). Primary tumor weight was not significantly changed across cohorts (Fig. 10). The treatment was well-tolerated, and animals maintained their body weight (Fig. 11). Dedicated veterinary histopathology review of 12 organ systems as well as standard clinical chemistry after three months of chronic, uninterrupted dosing of autochthonous KPC mice with 10 mg/kg metarrestin-infused chow (70 ppm) (see Table 6 below, "U" = undetermined) or two weeks of 25 mg/kg metarrestin via oral gavage failed to discern any histopathological or laboratory abnormalities in treated animals compared to control mice.

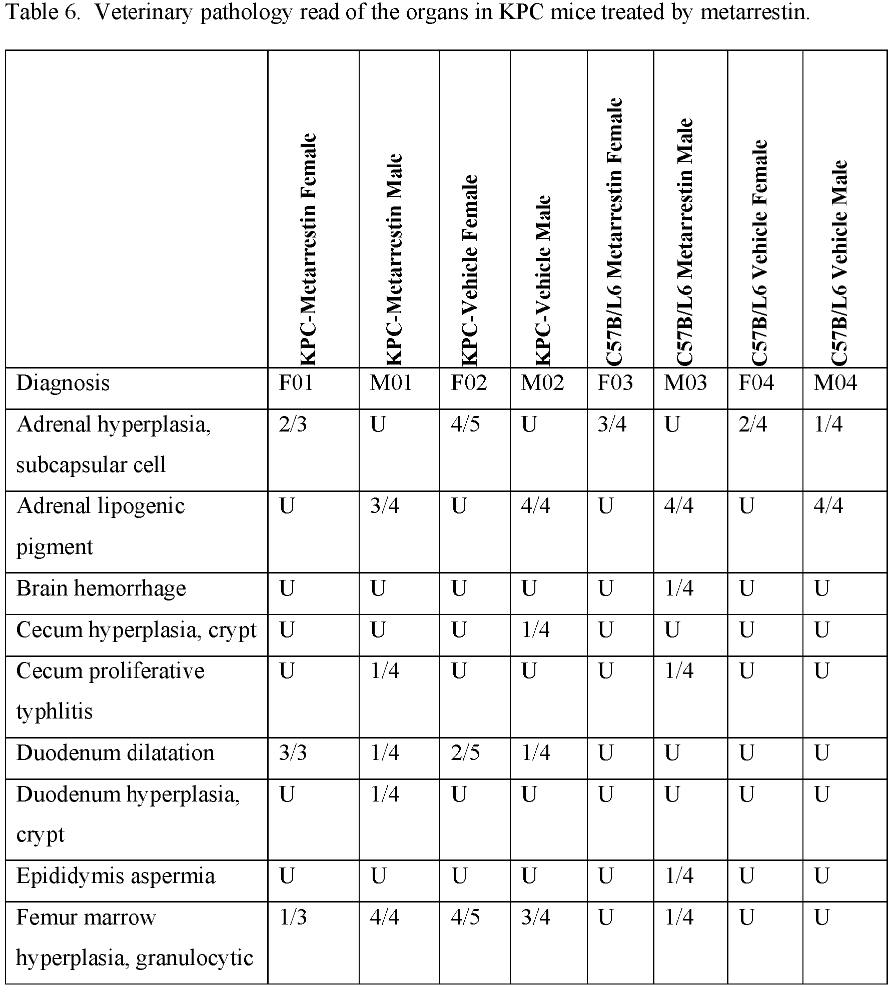

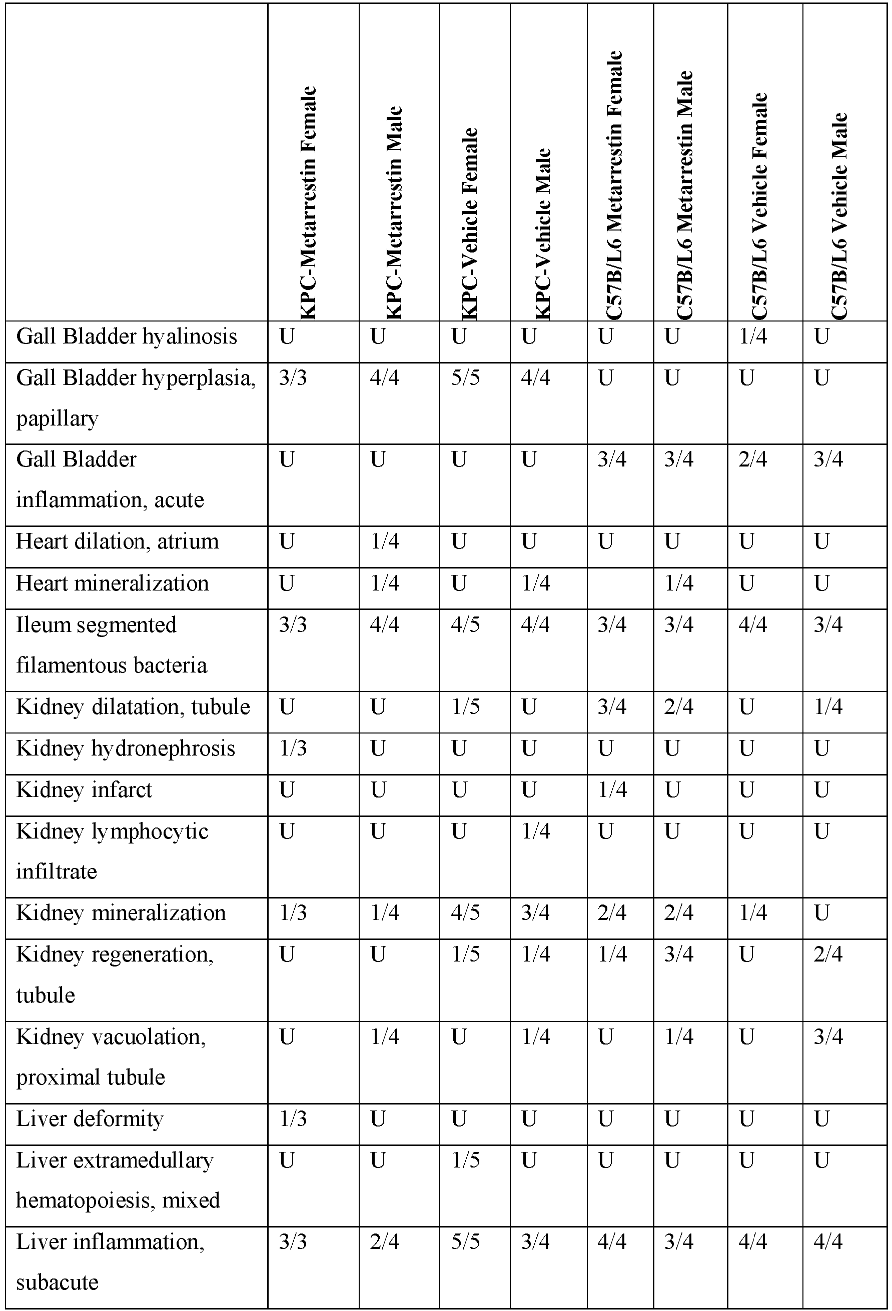

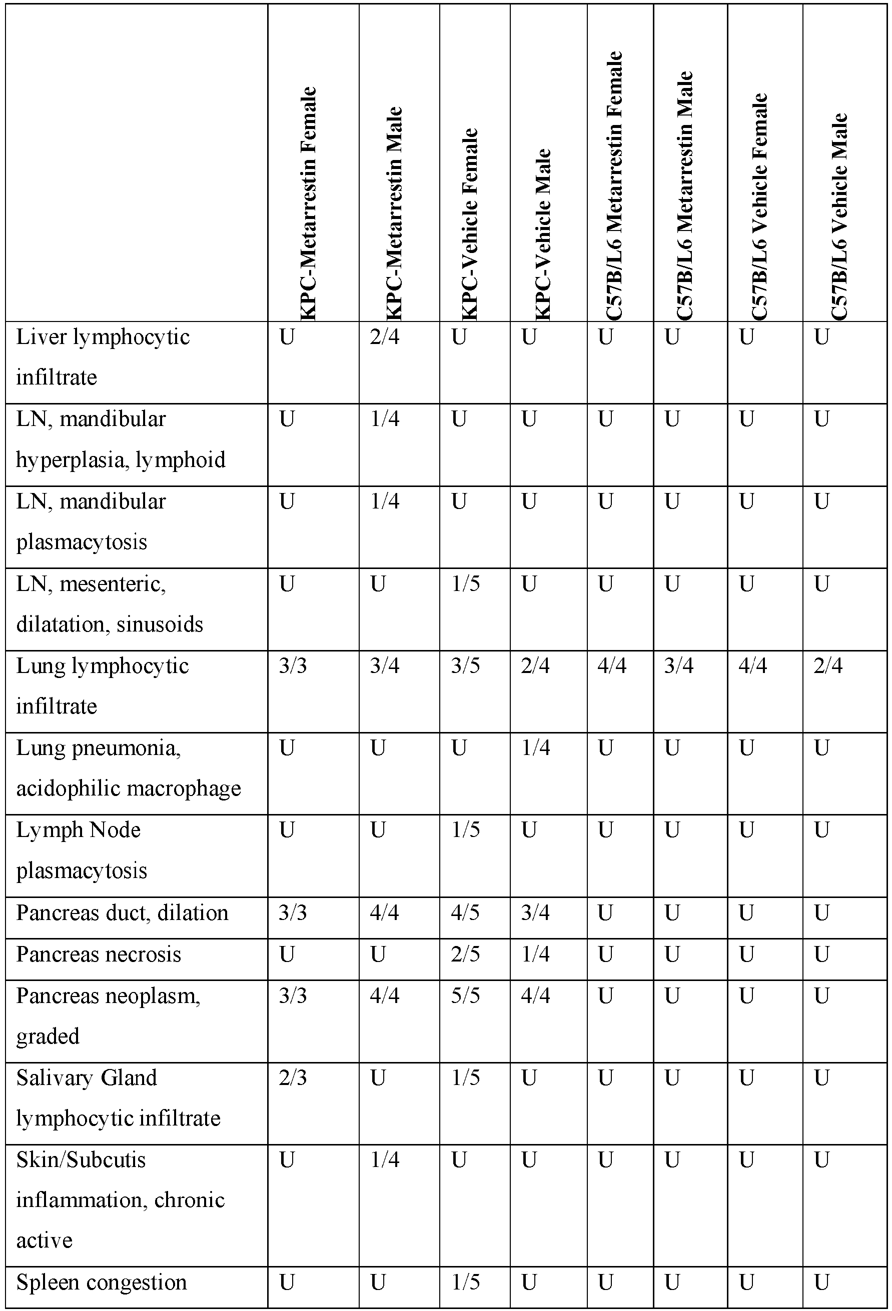

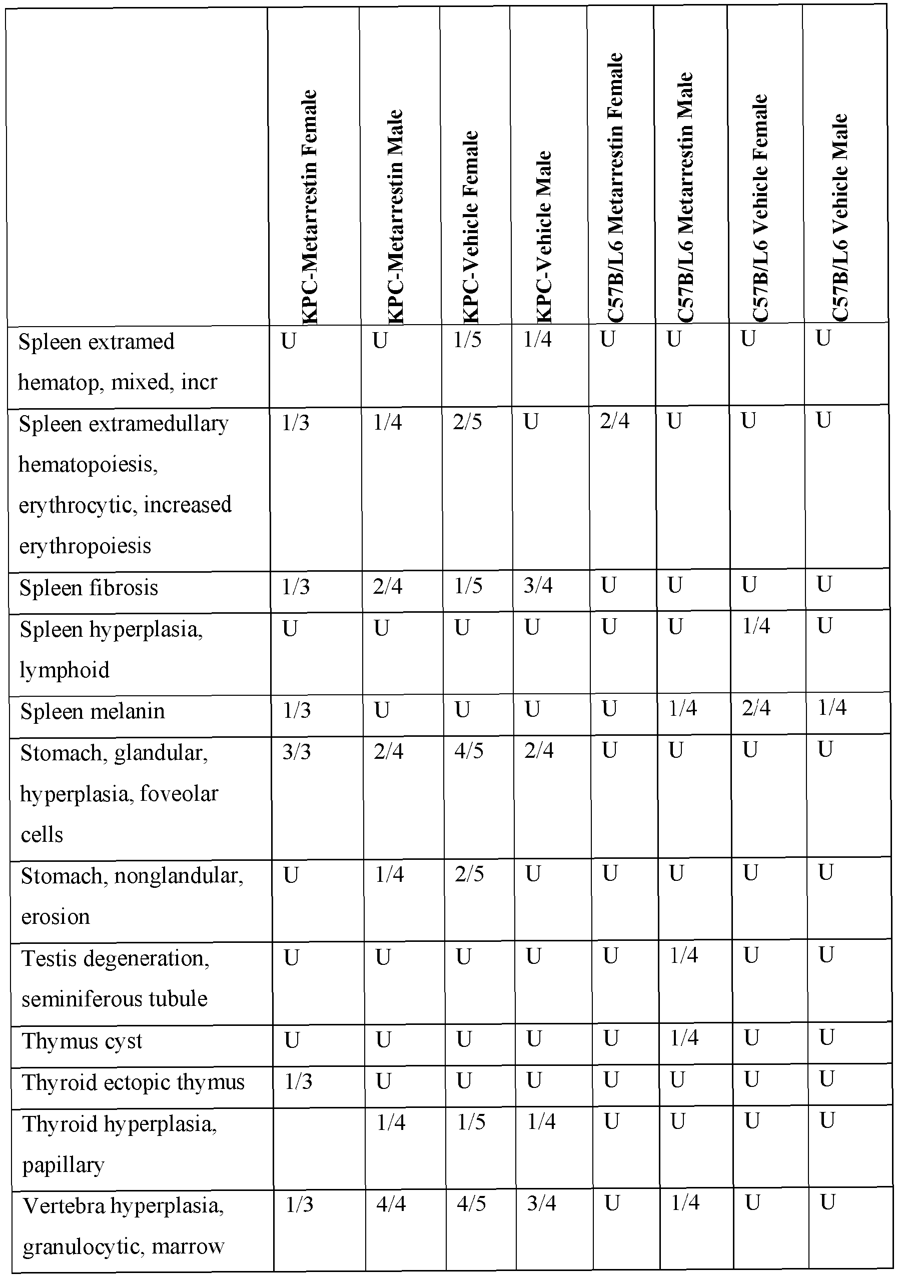

To determine whether PNCs in treated animals were impacted by metarrestin, PNC prevalence in primary tumors and metastatic lesions from control and metarrestin-treated animals (25 mg/kg) were examined 12 weeks after tumor inoculation. The results demonstrated a significant reduction of PNC prevalence (Fig. 2, 12 and 33, p < 0.01) in metastatic and primary tumor tissues, suggesting that PNC suppression is associated with the anti-metastatic activity of the compound. PNC prevalence in non-treated animals was higher in these groups of tumors and metastatic lesions than in those harvested at an earlier time (Fig. 6) or in cultured PANC1 cells (Fig. 2), consistent with previous findings that late-stage cancers have higher PNC prevalence.

To evaluate whether metarrestin treatment can provide a survival advantage by preventing metastatic progression, the above experiment was repeated, but mice were followed until death or when animals reached study endpoint. NSG mice were injected with 60,000 3D PANC1 cells (details of assay below), and daily treatment with metarrestin-infused chow (10 mg/kg; 70 ppm, micronized particles added to NIH 31 Haslan rodent diet) began 6 weeks after tumor cell implantation, when metastasis was limited to micrometastasis by histopathological examination (Fig. 63). Animals in the control group receiving regular chow started to die 25 days after the start of treatment, whereas the metarrestin treatment group had no mortality more than 90 days after the start of treatment (Fig. 13).

To evaluate whether metarrestin treatment impacts survival of mice with further evolved metastasis, NSG mice were injected with 60,000 3D PANC1 cells. After macrometastasis was visible on the liver surface, animals were randomized to receive metarrestin-infused chow (10 mg/kg; 70 ppm) or vehicle diet. Mice on the vehicle diet began to die within the first week of the treatment course (Fig. 14). Metarrestin treatment extended median overall survival by more than two-fold over the control group. Full necropsy at the study endpoint revealed significantly greater metastatic disease burden (p < 0.05) in the vehicle-treated animals (Fig. 15 and 16). Most animals in the control group showed near-complete or complete organ replacement with tumors, particularly in the liver and to a lesser degree in the lungs, whereas metarrestin-fed mice had preserved organs (Fig. 15). There was no significant difference in primary tumor growth in the pancreas between treated and untreated animals (Fig. 16). These findings suggest a survival gain due to suppression of metastasis-related death in metarrestin-treated mice.

Metarrestin was tested in PC3M xenograft mice to determine whether metarrestin's anti-metastasis activity (observed in the NSG PANC1 mice and *in vitro* pan-cancer PNC suppression) translates into anti-metastasis activity in additional cancer models. Two weeks after subcutaneous implantation of PC3M cells, mice were started on 5 mg/kg or 25 mg/kg metarrestin or vehicle by daily IP injection for 4 additional weeks. Tumor progression was tracked by both *in vivo* imaging systems ("IVIS") spectroscopy and tumor volume. Metastasis to the lungs was evaluated *ex vivo* through IVIS spectroscopy and by histopathology at the experimental endpoint. Metarrestin treatment at 25 mg/kg (P < 0.05) decreased development of lung metastasis compared to vehicle-treated mice (Fig. 17) and modestly reduced growth of primary tumor xenografts (Fig. 18). Body weight and behavior of the treated animals were not significantly different from control animals (Fig. 19). To evaluate the effect of metarrestin on a malignant xenograft considered closest to in vivo conditions, a patient-derived breast cancer xenotransplantation model (PDX) was used. Metastatic breast cancer cells harvested from the pleural fluid of a stage IV ductal breast cancer patient were inoculated into mice, and third generation mouse-passaged tumors were transplanted into the mammary fat pad of NOD.Cg*-Prkdc^{scid}Il2rg^{tm1Sug}*/JicTac mice. Treatment with metarrestin started after the tumors reached 150-200 mm³. Metarrestin (25 mg/kg) or vehicle was administered daily by IP injection for 4 weeks with a 5-day on and 2-day off schedule. Tumor size and total body weight were measured twice a week, and tumor weight was measured at experimental end-point.

The results show that metarrestin effectively inhibited the PDX growth, which formed entirely from metastatic cells from a patient without passing through culture *in vitro* (Fig. 20). The treatment was also well-tolerated, without apparent impact on animal body weight and behavior (Fig. 4E).

Pancreatic cancer model: 60,000 luciferase-tagged PANC1 spheres were injected into the pancreas of Nod/IL2gamma (null) mice (obtained from NCI Mouse Repository, Frederick, MD; on ACUC-approved animal protocol SB-211-2). At 4 weeks after inoculation, mice were treated once daily with metarrestin (5 mg/kg or 25 mg/kg) or vehicle via IP injections, which extended out for 6 more weeks. Mice were injected with luciferin 5 minutes before being sacrificed by CO₂ euthanasia. Organs were then individually dissected, subjected to quantitative xenogene imaging, and fixed with 4% non-buffered formaldehyde. Impact of metastatic burden was calculated and graphed as liver and lung/primary tumor ratios based on a five-minute photon count for the liver normalized to liver weight (g), which was then compared to the average photon count per weight (g) of the primary pancreatic tumor of the same animal. This calculation corrects for variations in luciferin injection, mouse heart rate, and perfusion. For overexpression of eEF1A2, 6×10^4 PANC1 3D spheres transduced with eEF1A2 (eEF1A2 OE) were injected into the tail of the pancreas of NSG mice. Mice of both groups were harvested at 6 weeks after implantation and subjected to necropsy. Liver metastasis/mm² was calculated as the sum of the volumes of the individual metastases. Tumor volume (mm³) was calculated as (L × W₂), where L = length (mm) and W = width (mm). Animal survival was measured from the first day of treatment until death. Animals in control and metarrestin treatment cohorts were allowed to progress under continuous treatment conditions until they reached a predetermined study end-point (15% weight loss, recognizable signs of morbidity, general lack of reflexes, abnormal posture, loss of ability to ambulate, labored respiration, inability to drink or feed) where, in order to prevent animal suffering, animals were euthanized in accordance with local animal care guidelines. Only "warm" necropsy specimens (blood, primary tumor, liver, and lungs) were used for PK analysis. All applicable institutional guidelines for the care and use of animals were followed.

Breast cancer model: The model derived from 0.2 liter of pleural effusion from a breast cancer patient (model #373342). These cancer cells had metastasized to the lung pleura from the initial tumor detected in the breast. The effusion was centrifuged at 5000 RPM for 10 minutes. The supernatant was removed, and the cells were inoculated with MATRIGEL gelatinous protein mixture into a mouse mammary fat pad. After the size of the tumor reached 1 cm, a 2 x 2 mm fragment of mammary fat pad was retransplanted into a mammary fat pad of another mouse. The tumors used in this experiment went through 4 passages. NOD.Cg*-Prkdc^{scid}Il2rg^{tm1Sug}*/JicTac strain of mice from Taconic was used in the experiment. The animals were acclimated up to 7 days before tumor inoculation. The 2 x 2 mm tumor was inoculated into the mammary fat pad. The study started after the tumor reached 150-200 mm³. Doses of 25 mg/kg metarrestin in 5% NMP, 20% PEG400, and 75% of 10% 2-hydroxypropyl-β-cyclodextrin (HP-β-CD) in water were injected IP 5 days a week for 4 weeks. Tumor size and total body weight were measured twice a week.

Prostate cancer model: Mice were implanted in the flank with 3 x 10₆ human PC-3M-luc-C6 pancreatic tumor cells (Caliper Life Sciences) and split into 4 groups of 10 mice. Dosing with 5 or 25 mg/kg drug or vehicle alone (negative control) was initiated after 2 weeks and continued until the experimental endpoint, 6 weeks after implantation. Primary tumor size (measured with calipers), live tumor cell content (measured by biophotonic imaging), and total body weight were determined each week. At the endpoint, animals were euthanized, metastatic organs removed, and metastases quantitated by biophotonic imaging of the organs.

### EXAMPLE 5

This example demonstrates that metarrestin treatment disrupts nucleolar structure and inhibits Pol I transcription.

To address the mechanisms by which metarrestin inhibits metastasis, cellular changes after metarrestin treatment were observed. Primary antibodies: 1:300 dilution for SH54 (anti-PTB antibody); 1: 300 for Nopp140 (anti-human Nopp140 rabbit polyclonal serum RS8); 1:50 for UBF (F-9) (Santa Cruz Biotechnology); 1:100 for RPA194 (Santa Cruz Biotechnology); 1:100 for CUG-BP1 (3B1) (Santa Cruz Biotechnology); 1:10 for fibrillarin (Sigma Cat #: ANA-N); 1:1000 for SC35; 1:300 for paxillin (ab32084); 1:600 for vinculin (Sigma V9131). The fluorescein-conjugated secondary antibodies (Jackson ImmunoResearch) and ALEXA FLUOR antibodies (Thermofisher Scientific) were used at a dilution of 1:200. Samples were visualized on a Nikon ECLIPSE Ti-E inverted fluorescence microscope using NIS-Elements AR 3.2 software (Nikon).

At 1 µM concentration, metarrestin induced collapse of the nucleolus from the typically integrated three substructures (arrows in Fig. 47): fibrillar centers (FC), dense fibrillar components (DFC), and granular components (GC), resulting in a segregation of the fibrillar from the granular components in the nucleus as observed by electron microscopy (EM) (Fig. 21A-21C, lower panels). The nucleolar disruption was reversible within 48 hours upon withdrawal of metarrestin (Fig. 47). Similar disruptions were observed among other treated cell lines, including PANC1 and PC3M cells (Fig. 48), primary tumor, and liver metastatic tissues in the PANC1 xenograft model (Fig. 21, right panels). Quantitative evaluation of the EM images demonstrated that nucleolar volume was significantly reduced (p < 0.0001) in the treated cell lines and cancer tissues (Fig. 22 and 49A-49C), but was not changed in treated normal mouse hepatocytes (Fig. 49C). The segregation of the nucleolar compartments observed by EM is believed to correspond to the nucleolar capping structures detected by immunofluorescence of nucleolar proteins (Fig. 21A-21C, 23, and 50). The coupling of nucleolar segregation with PNC loss was confirmed across several cell lines by immunolabeling with antibodies against the nucleolar Pol I transcription initiation factors, UBF (upstream binding transcription factor) (Fig. 23 and 50), or Pol I polymerase, RPA194 (Fig. 51). Factors involved in ribosome maturation, such as fibrillarin or NOPP140 (Fig. 51 and 52) were similarly reorganized. In contrast, UBF distribution was not changed when the normal human fibroblast GM02153 cell line was treated with metarrestin at the same concentration (Fig. 53).

To examine whether the disruption of the nucleolar structure impacts ribosome synthesis, a cell line expressing inducible GFP-RPL29 (a ribosomal subunit) was used. GFP-RPL29 induced by the addition of tetracycline (Fig. 24) showed similar cellular distribution to the endogenous ribosomal subunit, localized to the nucleolus and the cytoplasm as ribosomes assemble and traffic into the cytoplasm (Fig. 24). When GFP-RPL29 expression was induced after exposure to metarrestin for 5 hours (PNCs were disassembled and nucleolar structure altered), the production of GFP-RPL29 (Fig. 55 and 62) and its nucleolar localization appeared unchanged (Fig 24, right panel) in spite of the alterations in nucleolar structure (Fig. 54), indicating that metarrestin did not broadly impact Pol II transcription, translational, and nuclear import functions in treated cells within this time frame. However, the newly synthesized GFP-RPL29 was largely undetected in the cytoplasm of treated cells (Fig. 24, right panels, Fig. 54, lower panels), indicating a failure of the newly synthesized ribosomal protein to be incorporated into ribosomal particles and to be exported to the cytoplasm, which is a phenotype of ribosome synthesis defect. These findings show that metarrestin induces nucleolar ultrastructural disruption *in vitro* and *in vivo* with alterations of cellular distribution of ribosomal subunits, suggesting the involvement of ribosome synthesis modulation in the mechanism of action of the compound.

### EXAMPLE 6

This example demonstrates that metarrestin treatment reduces pre-RNA synthesis and Pol I occupancy at rDNA promoters.

To determine which steps in ribosomal biogenesis that are disrupted by metarrestin, rDNA transcription was evaluated using a BrU incorporation assay, an *in situ* run-on assay that detects the localization pattern of newly synthesized RNA. Cells were rinsed once in glycerol buffer (20 mM Tris-HCl pH 7.4, 5 mM MgCl₂, 25% glycerol, 0.5 mM PMSF, 0.5 mM EGTA) and followed by Br-U incorporation assay protocol. The cells were co-immunolabeled with C23 (Santa Cruz Biotechnology) at a 1:100 dilution and anti-BrdU that also recognizes Br-U (Sigma) at a 1:50 dilution.

A five-minute incubation of semi-permeabilized cells with a transcription cocktail containing BrU (Fig. 25) showed that the organization of newly synthesized rRNA (reflecting transcription sites) was altered into tight small clusters and nucleolar structure was disrupted, as represented by the dispersion of C23/nucleolin, a protein involved in many aspects of nucleolar function, from nucleoli (Fig. 25). To evaluate the impact on rDNA transcription in metarrestin-treated cells, the amounts of 5'ETS RNA were quantified. The amount of 5'ETS correlates with the amount of rDNA transcription because 5'ETS in nascent pre-rRNA transcripts is rapidly removed. Quantitative RT-PCR of 5'ETS in metarrestin-treated and DMSO-treated control cells showed a substantial reduction of 5'ETS RNA in metarrestin-treated cells (Fig. 26).

*ChIP-qPCR:* 2-5×10₇ cells were used for each ChIP assay according to previously described protocols. Briefly, HeLa cells with or without metarrestin treatment were crosslinked with 1% formaldehyde for 10 min at room temperature with rotation, and then crosslinking was quenched by the addition of glycine. Fixed chromatin was sonicated by Covaris and used for immunoprecipitation with the indicated antibody. Isolated DNA was analyzed by qPCR using SYBR green on CFX Connect Real-Time PCR Detection System (BioRad). The comparative cycle threshold method was applied to evaluate occupancy from replicate PCR reactions relative to the level of input. The primer sets used in the study included: rDNA promoter (F: 5'-GCT GCG ATG GTG GCG TTT TTG GGG (SEQ ID NO: 1) and R: 5'-ATA TAA CCC GGC GGC CCA AAA TTG CC) (SEQ ID NO: 2), 5'ETS (F: 5'-CGTGCCTGAGGTTTCTCC (SEQ ID NO: 3) and R: 5'-CCACCAACGGACGTGAAG (SEQ ID NO: 4)), 5.8S (F: 5'- GCA GGA CAC ATT GAT CAT CGA CAC (SEQ ID NO: 5) and R: 5'- GCG CGG CGG CAA GAG GAG (SEQ ID NO: 6)), 28S (F: 5' GGAGGAAAAGAAACTAACCAGGAT (SEQ ID NO: 7) and R: 5' GCCTCGATCAGAAGGACTTG (SEQ ID NO: 8)), and *U12* (F: 5'GATCTGCCCGACCTTATTCA (SEQ ID NO: 9) and R 5'AAACGCATTCACCACCTACC (SEQ ID NO: 10)).

RT- PCR: Total RNA was isolated from cells using TRIZOL reagent (Invitrogen) according to the manufacturer's instructions. cDNA was synthesized with Random Hexamer (IDT DNA Technologies) or gene-specific primers using M-MLV Reverse Transcriptase (Invitrogen 28025-013). The DNA was PCR-amplified with gene-specific primers. 5'ETS-F: CCTCCAGTGGTTGTCGACTT (SEQ ID NO: 11); 5'ETS-R: GAACGACACACCACCGTTC (SEQ ID NO: 12); eEF1A1-F: AACATTGTCGTCATTGGACA (SEQ ID NO: 13; eEF1A1-R: TTGATCTTTCCCTTTCTGGT (SEQ ID NO: 14); eEF1A2-F: 5'CCATGTGTGTGGAGAGCTTCTC (SEQ ID NO: 15); eEF1A2-R: 5' TCTCCACGTTCTTGATGACGCC (SEQ ID NO: 16; GAPDH-F: 5'ACCACAGTCCATGCCATCAC (SEQ ID NO: 17); GAPDH-R: 5'TCCACCACCCTGTTGCTGTA (SEQ ID NO: 18). The PCR products were resolved on a 1-2% agarose gel. Real time PCR was performed with ABI PRISM 7900HT instrument and Power SYBR Green PCR Master Mix (Life Technologies Cat# 4367659) at the Northwestern University Core Facility.

To examine the mechanisms by which metarrestin disrupts Pol I transcription, the overall state of the transcription machinery and rDNA chromatin structure in treated cells was evaluated.

Western blots were performed according to the manufacturer's protocols (Li-COR). Transfer membrane was from Millipore (cat. no. IPFL00010). The primary antibodies used were phospho- *γ*H2AX (Upstate 07-164, rabbit, 1:1000); p53 (Santa Cruz Biotechnology sc-6243 rabbit 1:500); UBF (F-9) (Santa Cruz Biotechnology, mouse, 1:500); RPA194 (Santa Cruz Biotechnology, sc-48385, mouse, 1:500); p53BP1 (Novus, NB100-304, rabbit 1:10,000); EFla (Millipore 05-235, mouse, 1:1000), HA-Tag (C29F4, Cell Signaling, rabbit 1:1000) and actin (Sigma A5060, rabbit, 1:3000, A4700, mouse, 1: 1000) as a loading control. LI-COR IRDye secondary antibodies (1:10,000), goat anti-mouse-680RD (926-68070), goat anti-mouse-800CW (926-32210), goat-anti-rabbit-680RD (926-68071), goat-anti-rabbit-800CW (926-32211) were used for visualization. Protein bands were detected using LI-COR Odyssey Image Studio (LI-COR Biosciences).

Western blot demonstrated that the amounts of Pol I large subunit RPA194 and UBF did not change significantly after 24 hours of treatment with metarrestin at 1 µM concentration in the three cell lines, PANC1, PC3M, and HeLa (Fig. 27). To examine whether an altered chromatin state of the rDNA clusters could be involved in the reduction of rRNA, psoralen-crosslinking experiments were carried out to determine the active/inactive rDNA chromatin ratios in metarrestin- and DMSO-treated cells. Psoralen crosslinks active accessible DNA under UV light without displacing nucleosomes or transcription factors, or changing chromatin states. Psoralen-crosslinked DNA moves slower on agarose gel and can be distinguished from the non-crosslinked DNA by Southern blots. The results showed that the ratio of active/inactive rDNA chromatin is similar in treated and untreated cells (Fig. 28), suggesting that rDNA chromatin is not grossly altered. To examine the interactions between Pol I transcription factors and rDNA, quantitative ChIP experiments were carried out using primer sets across promoter and coding regions of rDNA (Fig. 29-30E). Metarrestin treatment significantly reduced RPA194 occupancy on rDNA promoter and coding regions without significant impact on UBF binding (Fig. 30A-30E, p <0.01). As a control, a component of the small elongation complex, ICE1, did not show significant associations with rDNA sequences or altered association with its target gene *U12* transcribed by Pol II. These observations show that metarrestin impairs Pol I-rDNA interaction.

Genotoxic agents that intercalate into or alkylate DNA, such as actinomycin D, induce similar nucleolar segregation to that observed with metarrestin treatment. To determine whether metarrestin induces nucleolar changes through genotoxic effects or general cytotoxicity, the impact of metarrestin on DNA damage repair, cell cycle, and general Pol II transcription status in three cell lines, PANC1, PC3M, and HeLa, was evaluated. Cells were treated with 1 µM metarrestin or DMSO for 24 hours before fixation for flow cytometry, immunolabeling, or Western blot analyses. Evaluation of DNA damage response signature factors in treated cells (Fig. 56) demonstrated that neither phosphorylated yH2AX or p53BP1, nor phosphorylated p53 (HeLa and PC3M have little to no p53) were altered in these cell lines upon metarrestin treatment (Fig. 57A-57C). Cell cycle analyses of DNA content through flow cytometry did not show a significant alteration of cell cycle pattern upon metarrestin treatment at two different concentrations within 24 hours (Fig. 58). Evaluation of apoptotic index showed less than 1% of cells undergoing apoptosis in response to metarrestin treatment.

To further determine whether metarrestin interferes with Pol II transcription in general, CUGBP was immunolabeled. The steady state nuclear distribution of CUGBP, a multifunctional hnRNP protein highly enriched in the PNC, is dependent upon Pol II transcription. Selective inhibition of Pol II by α-amanitin induces a localization shift to the cytoplasm except for PNC-localized CUGBP (Fig. 59, top right panel). If metarrestin treatment significantly impacted Pol II transcription, one would expect a predominant cytoplasmic redistribution of CUGBP, as shown in α-amanitin-treated cells, however, that was not the case (Fig. 59, top middle panel). In addition, SC35, an essential pre-mRNA splicing factor normally has an intranuclear, interconnected speckle-distribution pattern (Fig. 59, lower left panel), which changed to isolated large dots in α-amanitin-treated cells (Fig. 59, lower right panel). In comparison, the SC35 pattern was not significantly changed in metarrestin-treated cells (Fig. 59, lower middle panel). Together with the observation that metarrestin treatment (Fig. 24) does not impact the induction of GFP-RPL29 expression (Fig. 54) and its nucleolar localization (Fig. 24 and 54), these findings suggest that metarrestin does not globally inhibit Pol II transcription, general protein synthesis, or nucleocytoplasmic trafficking within the effective concentration range, but has a selective effect on Pol I transcription and ribosome synthesis.

### EXAMPLE 7

This example demonstrates that reduction of RPA194 partially phenocopies the disruption of nucleoli and PNCs by metarrestin.

RNA interference: 75 nM Pol I1 Stealth siRNA (Invitrogen Cat#: 10620318) or Stealth negative control siRNA (Invitrogen Cat#: 12935-300) were transfected into cells with Lipofectamine RNAiMAX Transfection Reagent (Thermofisher Scientific) according to the manufacturer's instructions. The experiments were performed after 72 hours transfection. For eEF1A2 knockdown, 50 nM eEF1A2 DsiRNA (IDT Duplex pool HSC.RNAI.N001958.12/HSC.RNAI.N001958.12.2) or DS Scramble Negative Control siRNA (IDT) were transfected with Lipofectamine RNAiMAX as described above for 3 days. pcDNA3.1-HA-eEF1A2 plasmid was transfected after DsiRNA RNAi for 1 day with Lipofectamine 2000. The cells were then fixed for immunofluorescent labeling.

It was next determined whether metarrestin disassembles PNCs by inhibiting Pol I function and disrupting nucleoli. The large subunit of Pol I, RPA194, was knocked down by a specific siRNA, as evident by the reduction of the protein 72 hours after transfection with the siRNA oligos in cell lines PANC1, HeLa, and PC3M (Fig. 31). The reduction of RPA194 was sufficient to inhibit ribosome synthesis at a single cell level, as shown in HeLa cells expressing inducible GFP-RPL29, where RPA194 knockdown rendered cytoplasmic GFP-RPL29 near undetectable (Fig. 32, right panels). The reduction of RPA194 decreased PNC prevalence (Fig. 33), although not to the same extent as in metarrestin-treated cells (Fig. 2). Some PNCs also showed structural changes (Fig. 33), becoming crescent shaped around distorted nucleoli in RPA194-reduced cells (Fig. 34, top panels, arrows), similar to cells treated with metarrestin at a concentration that did not completely eliminate PNCs (Fig. 1, top, middle panel). The siRNA against RPA194 also disrupted the nucleolar structure into capping structure, as shown by labeling with anti-RPA194 for the residual RPA194 (Fig. 34, white arrows in the top 2nd panel). The changes in PNC and nucleoli in RPA194-silenced cells were similar to those observed with metarrestin treatment (Fig. 23 and 51). These data demonstrates that downregulating Pol I activity partially recapitulates the disruption of nucleolar and PNC structures induced by metarrestin treatment, and support the idea that metarrestin disrupts PNCs, at least in part, through inhibition of Pol I function.

### EXAMPLE 8

This example demonstrates that metarrestin binds the translation elongation factor eEFIA.

To identify the molecular target(s) of metarrestin and upstream factors involved in ribosomal and Pol I function, proteins that bind metarrestin through affinity purification using biotin-conjugated metarrestin (for structure and synthesis, see Fig. 65) were identified, which maintains its efficacy for PNC disassembly in cultured cells (Fig. 60). Competition studies using untagged metarrestin and proteomic analysis identified eukaryotic translation elongation factor (eEF1A) as a metarrestin-binding protein (Fig. 35). eEF1A has two isoforms, eEF1A1 and eEF1A2, and whereas eEF1A1 is ubiquitously expressed in all tissues, eEF1A2 is only expressed in brain, heart, and skeletal muscle, although its over- or re-expression, including in a stage-specific manner, has been described in a number of cancers, including pancreatic cancer. Binding of metarrestin to eEF1A was further confirmed in cells using a cellular thermal shift assay (CETSA), which showed an increase in the aggregation temperature of eEF1A upon metarrestin treatment (Fig. 36). However, the binding did not significantly change the amounts of the proteins, and western blot analyses showed that total eEF1A protein remained similar after 1 µM metarrestin treatment for 24 hours (Fig. 37).

eEF1As are multi-functional proteins, implicated in translation elongation, actin bundling, nuclear transport, and tRNA export. To evaluate whether eEF1A mediates the effect of metarrestin on cancer cells, eEF1A was either overexpressed or reduced through siRNA silencing. Whereas overexpression of HA-eEF1A1 or HA-EFF1A2 did not significantly increase total PNC prevalence (Fig. 38), overexpression of HA-eEF1A2, more so than HA-eEF1A1, increased the scattering patterns (number of PNCs per cell) of existing PNCs in PNC-containing cells (Fig. 38). Thus, eEF1A2 enhances PNC structures, but is alone not sufficient to induce significant formation of PNCs in PNC-negative cells. To evaluate the functional link between the phenotype of metarrestin-induced PNC disassembly and eEF1A2, PNC disassembly in wild type and eEF1A2-overexpressing cancer cells was analyzed. Cells with and without over expression of eEF1A2-HA (48 hours after transfection with an efficiency at around 70%, Fig. 61 and 64) were treated with metarrestin at 10 concentrations, and PNC prevalence was evaluated at 63× magnification. The results showed that increased expression of eEF1A2 increased the inhibitory concentration 50 (IC₅₀) of metarrestin for PNC disassembly (Fig. 39). To evaluate whether eEF1A2 indeed regulates the metastatic phenotype, PANC1 cells with (PANC1 eEF1A2 O.E.) or without (PANC1 control) overexpression of eEF1A2 were orthotopically injected into NSG mice. Six weeks after implantation, organs were subjected to histopathological evaluation. NSG PANC1 eEF1A2 O.E. mice showed a significantly increased metastatic disease burden compared to PANC1 control animals (Fig. 40A-40B, P <0.05), suggesting that metarrestin engages a target governing the metastatic phenotype.

To determine whether the reduction of eEF1A2 phenocopies metarrestin's impact on PNCs and nucleolar structure, eEF1A2 expression was reduced using siRNA oligos. Seventy-two hours after transfection, qRT-PCR showed a selective reduction of eEF1A2 (Fig. 41 and 42). To evaluate whether reduction of eEF1A2 impacts Pol I transcription, 5'ETS RNA expression was compared in cells with and without eEF1A2 knockdown, and the results showed a significant reduction (Fig. 43, P <0.01) of rDNA transcription. Correspondingly, immunofluorescence detection of nucleoli by anti-fibrillarin antibodies and of PNCs by SH54 showed that reduction of eEF1A2 disrupted nucleolar (nucleolar segregation) and PNC structure (loss or becoming elongated and crescent shaped, arrows in Fig. 44 and 45A-45B) similarly to metarrestin-treated (Fig. 1) or Pol I-knockdown cells (Fig. 34), although with lower efficacy compared to metarrestin (near 100% PNC disassembly and nucleolar distortion at 1 µM, Fig. 1, Fig. 23). The disruption could be partially rescued by overexpression of HA-eEF1A2 (Fig. 45, grey bars). Overall, these findings support the notion that eEF1A2 is involved in metastatic progression in pancreatic cancer and that interfering with its expression largely phenocopies the nucleolar and PNC structural alterations and Pol I inhibition induced by metarrestin, supporting the idea that eEF1A2, at least in part, mediates the nucleolar and PNC alterations induced by metarrestin treatment.

The above examples demonstrate, through *in vitro* assays and *in vivo* xenograft modeling, that metarrestin effectively inhibits PNC, cancer cell growth, cell invasion, tumor growth, and metastasis in pancreatic cancer models.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications recited in the claims appended hereto as permitted by applicable law.

## Claims

1. A pharmaceutical formulation comprising
(a) a compound of formula (I): 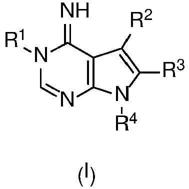
wherein R¹ is selected from the group consisting of 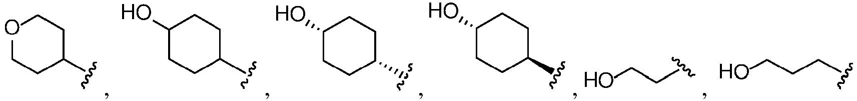 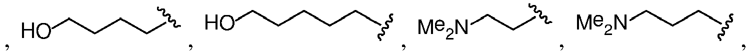 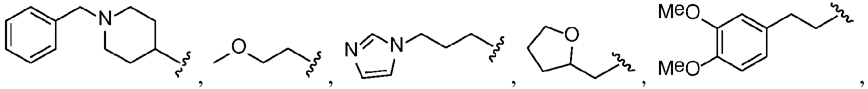 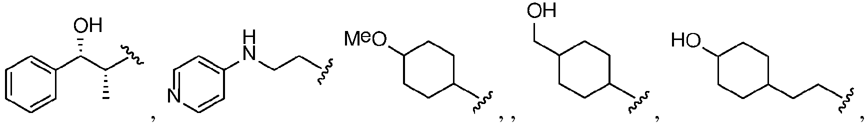 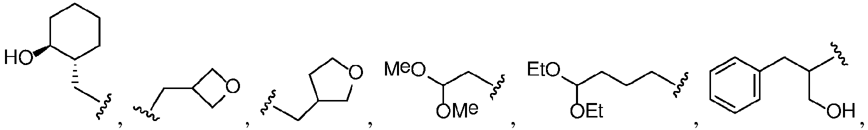 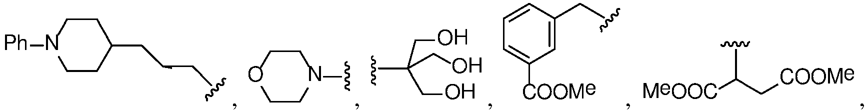 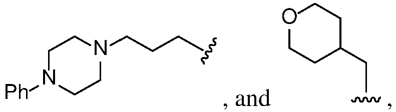
R² is phenyl,
R³ is phenyl,
R⁴ is benzyl,
or a pharmaceutically acceptable salt thereof,
and
(b) a pharmaceutically acceptable surfactant comprising one or more caprylocaproyl polyoxylglycerides and PEG-8 caprylic/capric glycerides,
further comprising caprylic acid.

2. The formulation of claim 1, wherein the compound of formula (I) is metarrestin.

3. The formulation of claim 1 or 2, for use in treating pancreatic adenocarcinoma in a mammal.

4. The formulation of claim 1 or 2 for the use of claim 3, further comprising a chemotherapeutic agent or a radiation treatment for the use of claim 3, optionally wherein the chemotherapeutic agent is gemcitabine.

5. The formulation of claim 1 or 2 for the use of claim 3 or 4, wherein the use results in any one or more of (I)-(V):
(I) the disrupting of a perinucleolar compartment in a cell in the mammal,
(II) reducing the prevalence of perinucleolar compartment in a cell in the mammal,
(III) reducing adenosine triphosphate (ATP) levels produced by metastatic cancer cells in the mammal,
(IV) reducing the colony formation of cancer cells in the mammal, and
(V) reducing the migration of cancer cells in the mammal.

6. The formulation of claim 1 or 2, for the use of any one of claims 3-5, wherein the mammal has stage I pancreatic adenocarcinoma, stage II pancreatic adenocarcinoma, stage **III** pancreatic adenocarcinoma, stage IV pancreatic adenocarcinoma, and/or metastatic pancreatic adenocarcinoma.

7. The formulation of claim 1 or 2, for the use of claim 6, wherein the chemotherapeutic agent is administered sequentially in any order with the compound of formula (I) or simultaneously with compound of formula (I).

8. The formulation of claim 1 or 2, for the use of any one of claims 3-7, wherein the formulation of claim 1, is administered after one or more pancreatic adenocarcinoma tumor(s) has been removed from the mammal.

9. The formulation of claim 1 or 2, for use in a method of treating pancreatic adenocarcinoma in a mammal, which method comprises:
(a) receiving a determination of a change in expression levels of one or both of FoxA1 and FoxO6, wherein the change is one or both of (i) the second determined level of FoxA1 expression being lower than the first determined level of FoxA1 expression and (ii) the second determined level of FoxO6 expression being higher than the first determined expression level of FoxO6;
(b) predicting the clinical response to the administration of the compound of formula (I); and
(c) administering the formulation of claim 1 or 2, to the mammal if the change is one or both of (i) the second determined level of FoxA1 expression being lower than the first determined level of FoxA1 expression and (ii) the second determined expression level of FoxO6 expression being higher than the first determined expression level of FoxO6 expression.

10. The formulation of claim 1 or 2 for the use of any one of claims 3-9, wherein the dose of the compound of formula (I) is from 0.1 to 80 mg/kg.

## Patentansprüche

1. Pharmazeutische Formulierung, umfassend
(a) eine Verbindung der Formel (I): 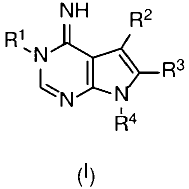
wobei R¹ ausgewählt ist aus der Gruppe bestehend aus 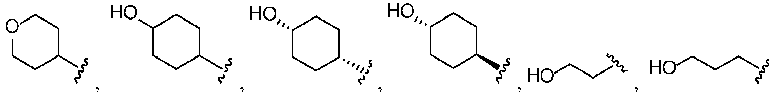 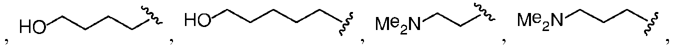 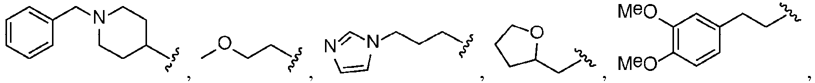 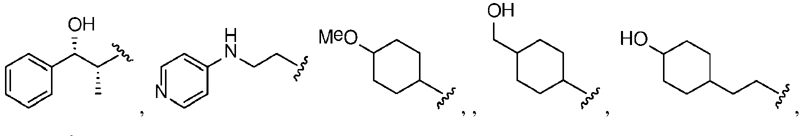 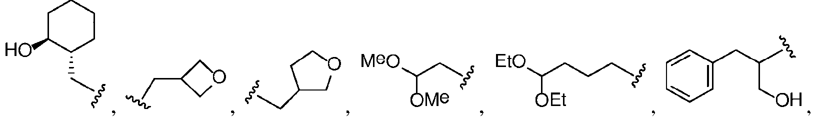 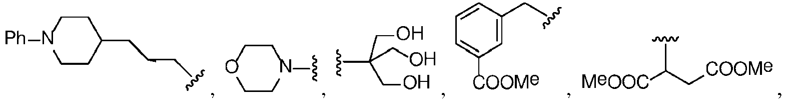 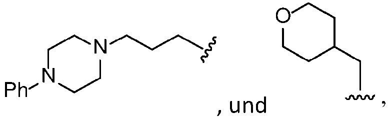
R² Phenyl ist,
R³ Phenyl ist
R⁴ Benzyl ist,
oder ein pharmazeutisch akzeptables Salz davon,
und
(b) ein pharmazeutisch akzeptables Tensid, das ein oder mehrere Caprylocaproylpolyoxylglyceride und PEG-8-Capryl-/Caprinsäureglyceride umfasst,
weiterhin umfassend Caprylsäure.

2. Formulierung nach Anspruch 1, wobei die Verbindung der Formel (I) Metarrestin ist.

3. Formulierung nach Anspruch 1 oder 2 zur Verwendung bei der Behandlung von Pankreasadenokarzinomen in einem Säugetier.

4. Formulierung nach Anspruch 1 oder 2 zur Verwendung nach Anspruch 3, ferner umfassend ein Chemotherapeutikum oder eine Strahlenbehandlung für die Verwendung von Anspruch 3, wobei das Chemotherapeutikum optional Gemcitabin ist.

5. Formulierung nach Anspruch 1 oder 2 zur Verwendung nach Anspruch 3 oder 4, wobei die Verwendung zu einem oder mehreren der folgenden Punkte (I) bis (V) führt:
(I) Stören eines perinukleolären Kompartiments in einer Zelle im Säugetier,
(II) Verringern der Prävalenz des perinukleolären Kompartiments in einer Zelle im Säugetier,
(III) Reduzieren des Adenosintriphosphat (ATP)-Levels, der von metastasierenden Krebszellen im Säugetier produziert wird,
(IV) Verringern der Koloniebildung von Krebszellen im Säugetier und
(V) Verringern der Migration von Krebszellen im Säugetier.

6. Formulierung nach Anspruch 1 oder 2 zur Verwendung nach einem der Ansprüche 3 bis 5, wobei das Säugetier an einem Pankreasadenokarzinom im Stadium I, einem Pankreasadenokarzinom im Stadium II, einem Pankreasadenokarzinom im Stadium III, einem Pankreasadenokarzinom im Stadium IV und/oder einem metastasierten Pankreasadenokarzinom leidet.

7. Formulierung nach Anspruch 1 oder 2 zur Verwendung nach Anspruch 6, wobei das Chemotherapeutikum in beliebiger Reihenfolge nacheinander mit der Verbindung der Formel (I) oder gleichzeitig mit der Verbindung der Formel (I) verabreicht wird.

8. Formulierung nach Anspruch 1 oder 2 zur Verwendung nach einem der Ansprüche 3 bis 7, wobei die Formulierung von Anspruch 1 verabreicht wird, nachdem ein oder mehrere Pankreasadenokarzinom-Tumor(e) aus dem Säugetier entfernt wurden.

9. Formulierung nach Anspruch 1 oder 2 zur Verwendung in einem Verfahren zur Behandlung von Pankreasadenokarzinomen in einem Säugetier, wobei das Verfahren umfasst:
(a) Empfangen einer Bestimmung einer Veränderung der Expressionslevel von einem oder beiden von FoxA1 und Fox06, wobei die Veränderung eines oder beide der folgenden Punkte umfasst: (i) das zweite bestimmte Level der FoxA1-Expression ist niedriger als das erste bestimmte Level der FoxA1-Expression und (ii) das zweite bestimmte Level der FoxO6-Expression ist höher als das erste bestimmte Expressionslevel von FoxO6;
(b) Vorhersagen des klinischen Ansprechens auf die Verabreichung der Verbindung der Formel (I); und
(c) Verabreichen der Formulierung gemäß Anspruch 1 oder 2 an das Säugetier, wenn die Veränderung eine oder beide der folgenden ist: (i) das zweite bestimmte Level der FoxA1-Expression ist niedriger als das erste bestimmte Level der FoxA1-Expression und (ii) das zweite bestimmte Expressionslevel der Fox06-Expression ist höher als das erste bestimmte Expressionslevel der Fox06-Expression.

10. Formulierung nach Anspruch 1 oder 2 zur Verwendung gemäß einem der Ansprüche 3 bis 9, wobei die Dosis der Verbindung der Formel (I) von 0,1 bis 80 mg/kg beträgt.

## Revendications

1. Formulation pharmaceutique, comprenant
(a) un composé de formule (1) : 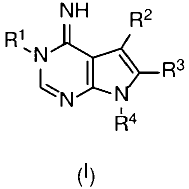
dans laquelle R¹ est choisi dans le groupe comprenant 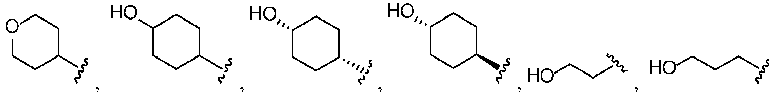 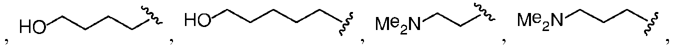 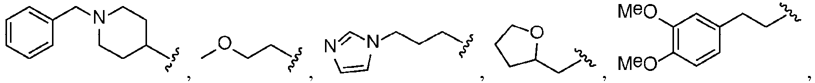 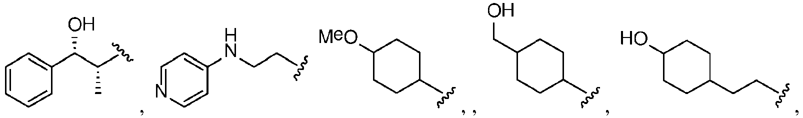 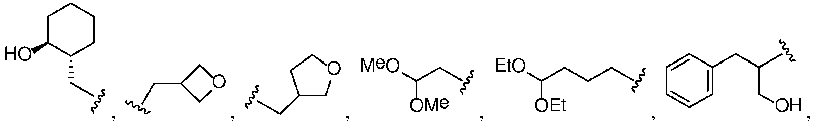 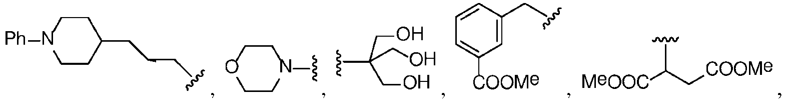 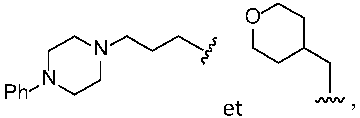
R² est un phényle,
R³ est un phényle,
R⁴ est un benzyle,
ou un sel pharmaceutiquement acceptable de celui-ci,
et
(b) un tensioactif pharmaceutiquement acceptable comprenant un ou plusieurs polyoxylglycérides de caprylocaproyle et glycérides de PEG-8 capryliques/capriques,
comprenant en outre de l'acide caprylique.

2. Formulation selon la revendication 1, dans laquelle le composé de formule (1) est la métarrestine.

3. Formulation selon la revendication 1 ou 2, pour utilisation dans le traitement d'un adénocarcinome pancréatique chez un mammifère.

4. Formulation selon la revendication 1 ou 2, pour l'utilisation selon la revendication 3, comprenant en outre un agent chimiothérapeutique ou un traitement par rayonnement pour l'utilisation selon la revendication 3, facultativement dans laquelle l'agent chimiothérapeutique est la gemcitabine.

5. Formulation selon la revendication 1 ou 2, pour l'utilisation selon la revendication 3 ou 4, dans laquelle l'utilisation a pour résultat une ou plusieurs parmi (I)-(V) :
(I) une perturbation d'un compartiment périnucléolaire dans une cellule du mammifère,
(II) une réduction de la prévalence du compartiment périnucléolaire dans une cellule du mammifère,
(III) une réduction de niveaux d'adénosine triphosphate (ATP) produits par des cellules cancéreuses métastatiques chez le mammifère,
(IV) une réduction de la formation de colonies de cellules cancéreuses chez le mammifère, et
(V) une réduction de la migration de cellules cancéreuses chez le mammifère.

6. Formulation selon la revendication 1 ou 2, pour l'utilisation selon l'une quelconque des revendications 3 à 5, dans laquelle le mammifère présente un adénocarcinome pancréatique de stade l, un adénocarcinome pancréatique de stade II, un adénocarcinome pancréatique de stade III, un adénocarcinome pancréatique de stade IV et/ou un adénocarcinome pancréatique métastatique.

7. Formulation selon la revendication 1 ou 2, pour l'utilisation selon la revendication 6, dans laquelle l'agent chimiothérapeutique est administré séquentiellement dans un ordre quelconque avec le composé de formule (I) ou simultanément avec le composé de formule (I).

8. Formulation selon la revendication 1 ou 2, pour l'utilisation selon l'une quelconque des revendications 3 à 7, dans laquelle la formulation selon la revendication 1 est administrée après qu'une ou plusieurs tumeurs d'adénocarcinome pancréatique aient été retirées du mammifère.

9. Formulation selon la revendication 1 ou 2, pour utilisation dans un procédé de traitement d'adénocarcinome pancréatique chez un mammifère, lequel procédé comprend les étapes consistant à :
(a) recevoir une détermination d'un changement de niveaux d'expression d'une ou des deux parmi FoxA1 et FoxO6, dans laquelle le changement est un ou les deux parmi (i) le second niveau déterminé d'expression de FoxA1 étant inférieur au premier niveau déterminé d'expression de FoxA1 et (ii) le second niveau déterminé d'expression de FoxO6 étant supérieur au premier niveau d'expression déterminé de FoxO6 ;
(b) prédire la réponse clinique à l'administration du composé de formule (I) ; et
(c) administrer la formulation de la revendication 1 ou 2, au mammifère si le changement est un ou les deux parmi (i) le second niveau déterminé d'expression de FoxA1 étant inférieur au premier niveau déterminé d'expression de FoxA1 et (ii) le second niveau déterminé d'expression de FoxO6 étant supérieur au premier niveau déterminé d'expression de FoxO6.

10. Formulation selon la revendication 1 ou 2 pour l'utilisation selon l'une quelconque des revendications 3 à 9, dans laquelle la dose du composé de formule (I) est de 0,1 à 80 mg/kg.
